# EUROPEAN PATENT SPECIFICATION

(11) **EP 2 539 355 B1**
(45) Date of publication and mention of the grant of the patent: **05.10.2016**
(21) Application number: 11748106.9
(22) Date of filing: 25.02.2011
(51) Int. Cl.: C07H 21/00, G01N 33/48, C12Q 1/68

(54) **IN-SITU HYBRIDIZATION WITH POLYTAG PROBES**
IN-SITU HYBRIDISIERUNG MIT POLYTAG-SONDEN
HYBRIDATION IN-SITU AVEC DES SONDES POLYTAG

(30) Priority: 26.02.2010 US 308670 P
(43) Date of publication of application: 02.01.2013
(73) Proprietor: Ventana Medical Systems, Inc., Tucson, Arizona 85755 (US)
(72) Inventor: FARRELL, Michael, Tucson Arizona 85704 (US); JIANG, Zeyu, Tucson Arizona 85737 (US); DAY, JR., William A., Tucson Arizona 85704 (US)
(74) Representative: Burger, Alexander
(86) International application number: PCT/US2011/026151
(87) International publication number: WO 2011/106583

(56) References cited:
- WO-A1-01/96608
- WO-A1-2011/038403
- WO-A2-01/94632
- WO-A2-2005/049848
- WO-A2-2007/001986
- WO-A2-2008/069884
- US-A1- 2003 059 786
- US-A1- 2006 172 314
- US-A1- 2008 220 979
- US-B2- 7 005 268

## Description

### FIELD OF THE INVENTION

The present invention provides probes and probe systems for detection of nucleic acids, and in particular probes and probe systems comprising target nucleic acid probes which comprise a plurality of detection target sequences and detection nucleic acid probes which hybridize to the detection target sequences of the target nucleic acid probes and which further comprise a plurality of detectable moieties, such as haptens.

### BACKGROUND OF THE INVENTION

Molecular cytogenetic techniques, such as fluorescence in situ hybridization (FISH), chromogenic in situ hybridization (CISH) and silver in situ hybridization (SISH), combine visual evaluation of chromosomes (karyotypic analysis) with molecular techniques. Molecular cytogenetics methods are based on hybridization of a nucleic acid probe to its complementary nucleic acid within a cell. A probe for a specific chromosomal region will recognize and hybridize to its complementary sequence on a metaphase chromosome or within an interphase nucleus (for example in a tissue sample). Probes have been developed for a variety of diagnostic and research purposes. For example, certain probes produce a chromosome banding pattern that mimics traditional cytogenetic staining procedures and permits identification of individual chromosomes for karyotypic analysis. Other probes are derived from a single chromosome and when labeled can be used as "chromosome paints" to identify specific chromosomes within a cell. Yet other probes identify particular chromosome structures, such as the centromeres or telomeres of chromosomes.

Unique sequence probes hybridize to single copy DNA sequences in a specific chromosomal region or gene. These are the probes used to identify the chromosomal critical region or gene associated with a syndrome or condition of interest. On metaphase chromosomes, such probes hybridize to each chromatid, usually giving two small, discrete signals per chromosome.

Hybridization of unique sequence probes has made possible detection of chromosomal abnormalities associated with numerous diseases and syndromes, including constitutive genetic anomalies, such as microdeletion syndromes, chromosome translocations, gene amplification and aneuploidy syndromes, neoplastic diseases as well as pathogen infections. Most commonly these techniques are applied to standard cytogenetic preparations on microscope slides. In addition, these procedures can be used on slides of formalin-fixed tissue, blood or bone marrow smears, and directly fixed cells or other nuclear isolates.

For example, these techniques are frequently used to characterize tumor cells for both diagnosis and prognosis of cancer. Numerous chromosomal abnormalities have been associated with the development of cancer (for example, aneuploidies such as trisomy 8 associated with certain myeloid disorders; translocations such as the BCR/ABL rearrangement in chronic myelogenous leukemia; and amplifications of specific nucleic acid sequences associated with neoplastic transformation). Molecular techniques can augment standard cytogenetic testing in the detection and characterization of such acquired chromosomal anomalies. For example, FISH has been used to look for early relapse and residual disease in nondividing cells. Immunocytochemical detection of cancer cells and FISH techniques have been combined to study chromosomal abnormalities in defined cell populations.

WO 2008/069884 A2 discloses two-stage amplification systems with a first non-enzymatic accumulation of an amplification oligomer that is the target substrate for a second nucleic amplification or assay. The two-stage amplification systems include one or more first solid supports to capture nucleic acids of interest in the first stage and a second solid support to capture so-called amplification oligomers, which result from the first stage amplification, as input target for the second stage.

WO 2007/001986 A2 discloses the detection of nucleic acid targets in fixed cells by an indirect labeling method. In the indirect labeling approach, the nucleic acid target molecule captures the label probe with a signal generating moiety through capture probes. In each capture probe, there is at least one section complementary to a section on the target molecule, and another section complementary to a section on the label probe.

WO 2005/049848 A2 discloses a method for hybridizing double-stranded DNA to a single stranded capture probe on a chip, wherein one end of the DNA is destabilized in the presence of Uracil-DNA glycosylase to create a single stranded section that can hybridize to the immobilized capture probe. WO 2005/049848 A2 further discloses several on-chip amplification methods to generate a larger and more detectable signal, the methods comprising one or more subsequent rolling circle amplification steps starting from the other end of the captured DNA.

US 7,005,268 discloses a method for detecting the presence of target molecules bound to a working electrode. In particular, US 7,005,268 discloses binding a target nucleic acid sequence to a probe immobilized on a substrate, and binding a labeling nucleic molecule with a photo-inducible charge-separation moiety to the bound target nucleic acid via complementary sequences in both the target and the labeling nucleic acid.

WO 01/94632 A2 discloses a method for in situ detection of a nucleic acid analyte of a known sequence, wherein cells are hybridized with a set of analyte-specific oligonucleotide probes ("target probes"), followed by hybridization with a series of oligonucleotide probes for signal amplification. Each series of oligonucleotides consists of a preamplifier probe, which hybridizes to the target probe, an amplifier oligonucleotide probe, which hybridizes to another portion of the preamplifier probe, and to which, in the last step of the series, a label probe is hybridized. I.e., when the nucleic acid analyte is present in the sample, the method of WO 01/94632 A2 results in an analyte-target probe-preamplifier probe-amplifier probe-label probe complex.The present disclosure provides improved probes and methods for producing such probes for use in diagnostic and research applications of in situ hybridization.

### SUMMARY OF THE INVENTION

Disclosed herein are probes and probe systems for detection of nucleic acids, and in particular probes and probe systems comprising target nucleic acid probes which comprise a plurality of detection target sequences and detection nucleic acid probes which hybridize to the detection target sequences of the target nucleic acid probes and which further comprise a plurality of detectable moieties, such as haptens. In some embodiments, the present disclosure provides nucleic acid molecules comprising: a target probe portion comprising a nucleic acid sequence complementary to a target nucleic acid sequence; and
a detection target portion comprising a plurality of sequences that are complementary to at least one detection probe sequence and non-complementary to the target nucleic acid sequence, the probe portion and the detection portion being in operable association. In some embodiments, the nucleic acid molecules are a nucleic acid selected from the group consisting of RNA and DNA. In some embodiments, the target probe portion of the nucleic acid molecule comprises nucleic acid analogs selected from the group consisting of LNA and PNA. In some embodiments, the plurality of sequences that are complementary to at least one detection probe sequence are repeated sequences that are substantially identical. In some embodiments, the detection target portion comprises greater than about 5 repeated sequences that are substantially identical. In some embodiments, the detection target portion comprises greater than about 10 repeated sequences that are substantially identical. In some embodiments, the repeated sequences that are substantially identical are from about 10 about 100 nucleotides in length. In some embodiments, the target probe portion is from about 10 to about 200 nucleotides in length. In some embodiments, the target probe portion is greater 99% complementary to the target nucleic acid sequence. In some embodiments, the target nucleic acid sequence is cellular target nucleic acid sequence. In some embodiments, the target nucleic acid sequence is a portion of a primary probe sequence. In some embodiments, the primary probe sequence comprises a portion complementary to a cellular target nucleic acid sequence and an adaptor portion which is not complementary to the cellular target nucleic acid sequence and the target probe portion of the nucleic acid molecule is complementary to the adaptor portion.

In some embodiments, the present disclosure provides systems for detection of a first target nucleic acid sequence comprising: a first nucleic acid molecule comprising, a first target probe portion comprising a nucleic acid sequence complementary to the first target nucleic acid sequence and
a first detection target portion comprising a plurality of first detection target sequences that are complementary to at least one detection probe nucleic acid sequence and non-complementary to the target nucleic acid sequence, and a second nucleic acid molecule comprising, a first detection probe portion complementary to the detection target sequences in the detection target portion of the first nucleic acid molecule and a first detectable moiety portion comprising at least one first detectable moiety either 5' or 3' to the detection probe portion. In some embodiments, the detectable moiety portion of the second nucleic acid molecule comprises a plurality of detectable moieties, wherein the detectable moieties are incorporated into the nucleic acid molecule. In some embodiments,the detectable moiety is directly detectable. In some embodiments, the detectable moiety is indirectly detectable. In some embodiments, the detectable moiety is selected from the group consisting of a signal-generating moiety and a first member of a pair of binding moieties. In some embodiments, the signal-generation moiety is selected from the group consisting of a quantum dot, a fluorophore, a fluorescent protein, an enzyme, and colloidal gold. In some embodiments, the first member of a pair of binding moieties is a hapten. In some embodiments, the hapten is selected from the group consisting of biotin, 2,4-Dintropheyl (DNP), Fluorescein deratives, Digoxygenin (DIG), 5-Nitro-3-pyrozolecarbamide (nitropyrazole, NP), 4,5,-Dimethoxy-2-nitrocinnamide (nitrocinnamide, NCA), 2-(3,4-Dimethoxyphenyl)-quinoline-4-carbamide (phenylquinolone, DPQ), 2,1,3-Benzoxadiazole-5-carbamide (benzofurazan, BF), 3-Hydroxy-2-quinoxalinecarbamide (hydroxyquinoxaline, HQ), 4-(Dimethylamino)azobenzene-4'-sulfonamide (DABSYL), Rotenone isoxazoline (Rot), (E)-2-(2-(2-oxo-2,3-dihydro-1H-benzo[b][1,4]diazepin-4-yl)phenozy)acetamide (benzodiazepine, BD), 7-(diethylamino)-2-oxo-2H-chromene-3-carboxylic acid (coumarin 343, CDO), 2-Acetamido-4-methyl-5-thiazolesulfonamide (thiazolesulfonamide, TS), and p-Mehtoxyphenylpyrazopodophyllamide (Podo). In some embodiments, the second nucleic acid molecule comprises at least 5 detectable moieties. In some embodiments, the second nucleic acid molecule comprises at least 10 detectable moieties. In some embodiments, the systems further comprise a specific binding agent that binds to the first member of a pair of binding moieties. In some embodiments, the specific binding agent comprises a specific binding moiety that binds to the first member of a pair of binding moieties and comprises a signal generating moiety. In some embodiments, the specific binding moiety is selected from the group consisting of avidin and an antigen binding molecule. In some embodiments, the antigen binding molecule is an antibody or fragment thereof. In some embodiments, the antibody or fragment thereof binds to a hapten. In some embodiments, the specific binding agent comprises a signal generating moiety selected from the group consisting of a quantum dot, a fluorophore, a fluorescent protein, an enzyme, and colloidal gold. In some embodiments, the second nucleic acid molecule is a nucleic acid selected from the group consisting of RNA and DNA. In some embodiments, the second nucleic acid molecule comprises nucleic acid analogs selected from the group consisting of LNA and PNA nucleotides. In some embodiments, the target nucleic acid sequence is a cellular target nucleic acid sequence. In some embodiments, the target nucleic acid sequence is a portion of a first primary probe sequence. In some embodiments, the first primary probe sequence comprises a portion complementary to a cellular target nucleic acid sequence and an adaptor portion which is not complementary to the cellular target nucleic acid sequence and the target probe portion of the nucleic acid molecule is complementary to the adaptor portion. In some embodiments, the systems further comprise at least third and fourth nucleic acid molecules, the third nucleic acid molecule comprising, a second target probe portion comprising a nucleic acid sequence complementary to a second target nucleic acid sequence and a second detection target portion comprising a plurality of second detection target sequences that are complementary to at least one detection probe nucleic acid sequence and non-complementary to the target nucleic acid sequence, and the fourth nucleic acid molecule comprising, a second detection probe portion complementary to the second detection target sequences in the second detection portion of the third nucleic acid molecule and
a second detectable moiety portion comprising at least one second detectable moiety either 5' or 3' to the to the detection probe portion. In some embodiments, the second target nucleic acid sequence is a second cellular target nucleic acid sequence. In some embodiments, the second target nucleic acid sequence is a portion of a second primary probe sequence. In some embodiments, the second primary probe sequence comprises a portion complementary to a cellular target nucleic acid sequence and an adaptor portion which is not complementary to the cellular target nucleic acid sequence and the target probe portion of the nucleic acid molecule is complementary to the adaptor portion.

The present invention provides methods according to claim 1, for detecting a first target nucleic acid sequence in a sample comprising: contacting the sample with a first nucleic acid molecule comprising a target probe portion comprising a nucleic acid sequence complementary to the first target nucleic acid sequence and a detection target portion comprising a plurality of first detection target sequences that are complementary to at least one detection probe nucleic acid sequence and non-complementary to the target nucleic acid sequence, under conditions where the detection target portion of the first nucleic acid molecule hybridizes to the target nucleic acid sequence; contacting the first nucleic acid molecule with a plurality of second nucleic acid molecules each comprising a detection probe portion complementary to the detection target sequences of the first nucleic acid molecule and a detectable moiety portion comprising at least one first detectable moiety either 5' or 3' to the detection probe portion, under conditions such that the detection probe portion of the second nucleic acid molecule hybridizes to the first detection target sequences of the first nucleic acid molecule; and detecting the at least one first detectable moiety. In some embodiments, the detecting is direct detection. In some embodiments, the detecting is indirect detection. In some embodiments, the sample is selected from the group consisting of a tissue sample, an organism sample, a sample on a solid substrate, and sample on a microtiter plate, and a sample on a magnetic particle. In some embodiments, the target nucleic acid sequence is selected from the group consisting of genomic DNA, nuclear DNA, RNA, mRNA, and cytoplasmic nucleic acid. In some embodiments, the target nucleic acid is isolated from a tissue or organism. In some embodiments, the detecting comprises detection selected from the group consisting of colorimetric, radiometric, fluorometric, and microscopic detection. In some embodiments, the detecting comprises contacting the sample with a specific binding agent that binds to the at least one detectable moiety on the second nucleic acid molecule. In some embodiments, the target probe portion of the first nucleic acid molecule and the target nucleic acid sequence and the detection probe portion of the second nucleic acid and the detection target sequences of the first nucleic acid molecule have melting points within about 10 degrees Celsius. In some embodiments, the target nucleic acid sequence is a cellular target nucleic acid sequence. In some embodiments, the target nucleic acid sequence is a portion of a primary probe sequence. In some embodiments,
the primary probe sequence comprises a portion complementary to a cellular target nucleic acid sequence and an adaptor portion which is not complementary to the cellular target nucleic acid sequence and the target probe portion of the nucleic acid molecule is complementary to the adaptor portion. In some embodiments, the methods further comprise contacting sample with a third nucleic acid molecule comprising a target probe portion comprising a nucleic acid sequence complementary to a second target nucleic acid sequence and a detection target portion comprising a plurality of second detection target sequences that are complementary to at least one detection probe nucleic acid sequence and non-complementary to the target nucleic acid sequence, under conditions where the target probe portion of the first nucleic acid molecule hybridizes to the target nucleic acid sequence; contacting the third nucleic acid molecule with a plurality of fourth nucleic acid molecules each comprising a detection probe portion complementary to the detection target sequences of the third nucleic acid molecule and a detectable moiety portion comprising at least one second detectable moiety either 5' or 3' to the detection probe portion, under conditions such that the detection probe portion of the fourth nucleic acid molecule hybridizes to the second detection target sequences of the third nucleic acid molecule; and detecting the at least one second detectable moiety. In some embodiments, the first and second target nucleic acid sequences are part of the same molecule. In some embodiments, the first and second detectable moieties are the same. In some embodiments, the second target nucleic acid sequence is a second cellular target nucleic acid sequence. In some embodiments, the second target nucleic acid sequence is a portion of a second primary probe sequence. In some embodiments, the second primary probe sequence comprises a portion complementary to a cellular target nucleic acid sequence and an adaptor portion which is not complementary to the cellular target nucleic acid sequence and the target probe portion of the nucleic acid molecule is complementary to the adaptor portion.

Disclosed herein are also kits comprising: a first nucleic acid molecule comprising a target probe portion comprising a nucleic acid sequence complementary to the target nucleic acid sequence and a detection target portion about greater than 200 nucleotides in length comprising a plurality of detection target sequences that are complementary to at least one detection probe nucleic acid sequence and non-complementary to the target nucleic acid sequence, and a second nucleic acid molecule comprising a detection probe portion complementary to the detection target sequences in the detection portion of the first nucleic acid molecule and a detectable moiety portion comprising at least one detectable moiety either 5' or 3' to the to the detection probe portion. In some embodiments, the kits further comprise a specific binding agent that binds to the at least one detectable moiety. In some embodiments, the specific binding agent comprises a specific binding moiety conjugated to a signal generating moiety. In some embodiments, the kits further comprise at least a second nucleic acid molecule comprising a second target probe portion and second detection target portion. In some embodiments, the target nucleic acid sequence is a cellular target nucleic acid sequence. In some embodiments, the target nucleic acid sequence is a portion of a primary probe sequence. In some embodiments, the primary probe sequence comprises a portion complementary to a cellular target nucleic acid sequence and an adaptor portion which is not complementary to the cellular target nucleic acid sequence and the target probe portion of the nucleic acid molecule is complementary to the adaptor portion.

### DESCRIPTION OF THE FIGURES

Figure 1 provides a schematic depiction of one embodiment of the invention.
Figure 2 is a dot blot stain for detection of 18s rRNA with three different PolyTag riboprobes.
Figures 3a and 3b are fluorescence micrographs of detection of actin mRNA.
Figure 4 is a micrograph of detection of the chromosome 17 centromere.
Figures 5 and 5b are a micrographs of the results of a SISH assay for detecting the PTEN gene with five-copy (5a) or ten-copy (5b) ssDNA PolyTag probes.
Figure 6 is a micrograph of the results of the results of a SISH assay for detecting the PTEN gene with a primary probe followed by hybridization with PolyTag probes.

### DEFINITIONS

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. The term "plurality" is used synonymously with the phrase "more than one," that is, two or more. It is further to be understood that all base sizes or amino acid sizes, and all molecular weight or molecular mass values, given for nucleic acids or polypeptides are approximate, and are provided for description. The term "comprises" means "includes." The abbreviation, "e.g.," is derived from the Latin exempli gratia, and is used herein to indicate a non-limiting example. Thus, the abbreviation "e.g.," is synonymous with the term "for example." Although methods and materials similar or equivalent to those described herein can be used in the practice or testing of this disclosure, suitable methods and materials are described below.

In order to facilitate review of the various embodiments of this disclosure, the following explanations of specific terms are provided:

Antibody: "Antibody" collectively refers to immunoglobulins or immunoglobulin-like molecules (including by way of example and without limitation, IgA, IgD, IgE, IgG and IgM, combinations thereof, and similar molecules produced during an immune response in any vertebrate, for example, in mammals such as humans, goats, rabbits and mice) and antibody fragments that specifically bind to a molecule of interest (or a group of highly similar molecules of interest) to the substantial exclusion of binding to other molecules (for example, antibodies and antibody fragments that have a binding constant for the molecule of interest that is at least 10³ M⁻¹ greater, at least 10⁴ M⁻¹ greater or at least 10⁵ M⁻¹ greater than a binding constant for other molecules in a biological sample.

More particularly, "antibody" refers to a polypeptide ligand comprising at least a light chain or heavy chain immunoglobulin variable region which specifically recognizes and binds an epitope of an antigen. Antibodies are composed of a heavy and a light chain, each of which has a variable region, termed the variable heavy (V_{H}) region and the variable light (V_{L}) region. Together, the V_{H} region and the V_{L} region are responsible for binding the antigen recognized by the antibody.

This includes intact immunoglobulins and the variants and portions of them well known in the art. Antibody fragments include proteolytic antibody fragments [such as F(ab')₂ fragments, Fab' fragments, Fab'-SH fragments and Fab fragments as are known in the art], recombinant antibody fragments (such as sFv fragments, dsFv fragments, bispecific sFv fragments, bispecific dsFv fragments, F(ab)'₂ fragments, single chain Fv proteins ("scFv"), disulfide stabilized Fv proteins ("dsFv"), diabodies, and triabodies (as are known in the art), and camelid antibodies (see, for example, U.S. Pat. Nos. 6,015,695; 6,005,079-5,874,541; 5,840,526; 5,800,988; and 5,759,808). A scFv protein is a fusion protein in which a light chain variable region of an immunoglobulin and a heavy chain variable region of an immunoglobulin are bound by a linker, while in dsFvs, the chains have been mutated to introduce a disulfide bond to stabilize the association of the chains. The term also includes genetically engineered forms such as chimeric antibodies (for example, humanized murine antibodies), heteroconjugate antibodies (such as, bispecific antibodies). See also, Pierce Catalog and Handbook, 1994-1995 (Pierce Chemical Co., Rockford, Ill.); Kuby, J., Immunology, 3.sup.rd Ed., W.H. Freeman & Co., New York, 1997.

Typically, a naturally occurring immunoglobulin has heavy (H) chains and light (L) chains interconnected by disulfide bonds. There are two types of light chain, lambda (.lamda.) and kappa (k). There are five main heavy chain classes (or isotypes) which determine the functional activity of an antibody molecule: IgM, IgD, IgG, IgA and IgE.

Each heavy and light chain contains a constant region and a variable region, (the regions are also known as "domains"). In combination, the heavy and the light chain variable regions specifically bind the antigen. Light and heavy chain variable regions contain a "framework" region interrupted by three hypervariable regions, also called "complementarity-determining regions" or "CDRs". The extent of the framework region and CDRs have been defined (see, Kabat et al., Sequences of Proteins of Immunological Interest, U.S. Department of Health and Human Services, 1991. The Kabat database is now maintained online. The sequences of the framework regions of different light or heavy chains are relatively conserved within a species. The framework region of an antibody, that is the combined framework regions of the constituent light and heavy chains, serves to position and align the CDRs in three-dimensional space.

The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a V_{H} CDR3 is located in the variable domain of the heavy chain of the antibody in which it is found, whereas a V_{L} CDR1 is the CDR1 from the variable domain of the light chain of the antibody in which it is found. An antibody that binds RET will have a specific V_{H} region and the V_{L} region sequence, and thus specific CDR sequences. Antibodies with different specificities (i.e. different combining sites for different antigens) have different CDRs. Although it is the CDRs that vary from antibody to antibody, only a limited number of amino acid positions within the CDRs are directly involved in antigen binding. These positions within the CDRs are called specificity determining residues (SDRs).

"Binding or stable binding" refers to the association between two substances or molecules, such as the hybridization of one nucleic acid molecule (e.g., a binding region) to another (or itself) (e.g., a target nucleic acid molecule). A nucleic acid molecule binds or stably binds to a target nucleic acid molecule if a sufficient amount of the nucleic acid molecule forms base pairs or is hybridized to its target nucleic acid molecule to permit detection of that binding.

A "binding region" is a segment or portion of a target nucleic acid molecule that is unique to the target molecule, and in some examples is free or substantially free of repetitive (or other undesired) nucleic acid sequence. The nucleic acid sequence of a binding region and its corresponding target nucleic acid molecule have sufficient nucleic acid sequence complementarity such that when the two are incubated under appropriate hybridization conditions, the two molecules will hybridize to form a detectable complex. A target nucleic acid molecule can contain multiple different binding regions, such as at least 10, at least 50, at least 100, or at least 1000 unique binding regions. In particular examples, a binding region is typically several hundred to several thousand base pairs in length. However, in some examples a binding region is shorter, such as 50 to 200 base pairs in length. When obtaining binding regions from a target nucleic acid sequence, the target sequence can be obtained in its native form in a cell, such as a mammalian cell, or in a cloned form (e.g., in a vector).

A nucleic acid molecule is said to be "complementary" with another nucleic acid molecule if the two molecules share a sufficient number of complementary nucleotides to form a stable duplex or triplex when the strands bind (hybridize) to each other, for example by forming Watson-Crick, Hoogsteen or reverse Hoogsteen base pairs. Stable binding occurs when a nucleic acid molecule remains detectably bound to a target nucleic acid sequence (e.g., genomic target nucleic acid sequence) under the required conditions.

Complementarity is the degree to which bases in one nucleic acid molecule (e.g., target nucleic acid probe) base pair with the bases in a second nucleic acid molecule (e.g., genomic target nucleic acid sequence). Complementarity is conveniently described by percentage, that is, the proportion of nucleotides that form base pairs between two molecules or within a specific region or domain of two molecules.

In the present disclosure, "sufficient complementarity" means that a sufficient number of base pairs exist between one nucleic acid molecule or region thereof and a target nucleic acid sequence (e.g., genomic target nucleic acid sequence) to achieve detectable binding. A thorough treatment of the qualitative and quantitative considerations involved in establishing binding conditions is provided by Beltz et al. Methods Enzymol. 100:266-285, 1983, and by Sambrook et al. (ed.), Molecular Cloning. A Laboratory Manual, 2nd ed., vol. 1-3, Cold Spring Harbor Laboratory Press, Cold Spring Harbor, N.Y., 1989.

A "computer implemented algorithm" is an algorithm or program (set of executable code in a computer readable medium) that is performed or executed by a computing device at the command of a user. In the context of the present disclosure, computer implemented algorithms can be used to facilitate (e.g., automate) selection of polynucleotide sequences with particular characteristics, such as identification of repetitive (or other undesired, e.g., background producing) nucleic acid sequences or unique binding regions of a target nucleic acid sequence. Typically, a user initiates execution of the algorithm by inputting a command, and setting one or more selection criteria, into a computer, which is capable of accessing a sequence database. The sequence database can be encompassed within the storage medium of the computer or can be stored remotely and accessed via a connection between the computer and a storage medium at a nearby or remote location via an intranet or the internet. Following initiation of the algorithm, the algorithm or program is executed by the computer, e.g., to select one or more polynucleotide sequences that satisfy the selection criteria. Most commonly, the selected polynucleotide sequences are then displayed (e.g., on a screen) or outputted (e.g., in printed format or onto a computer readable medium).

The terms "conjugating, joining, bonding or linking" refer to covalently linking one molecule to another molecule to make a larger molecule. For example, making two polypeptides into one contiguous polypeptide molecule, or to covalently attaching a hapten or other molecule to a polypeptide, such as an scFv antibody. In the specific context, the terms include reference to joining a specific binding molecule such as an antibody to a signal generating moiety, such as a quantum dot. The linkage can be either by chemical or recombinant means. "Chemical means" refers to a reaction between the antibody moiety and the effector molecule such that there is a covalent bond formed between the two molecules to form one molecule.

The term "coupled", when applied to a first atom or molecule being "coupled" to a second atom or molecule can be both directly coupled and indirectly coupled. A secondary antibody provides an example of indirect coupling. One specific example of indirect coupling is a rabbit anti-hapten primary antibody that is bound by a mouse anti-rabbit IgG antibody, that is in turn bound by a goat anti-mouse IgG antibody that is covalently linked to a detectable label.

The term "corresponding" in reference to a first and second nucleic acid (for example, a binding region and a target nucleic acid sequence) indicates that the first and second nucleic acid share substantial sequence identity or complementarity over at least a portion of the total sequence of the first and/or second nucleic acid. Thus, a binding region corresponds to a target nucleic acid sequence if the binding region possesses substantial sequence identity or complementarity (e.g., reverse complementarity) with (e.g., if it is at least 80%, at least 85%, at least 90%, at least 95%, or even 100% identical or complementary to) at least a portion of the target nucleic acid sequence. For example, a binding region can correspond to a target nucleic acid sequence if the binding region possesses substantial sequence identity to one strand of a double-stranded target nucleic acid sequence (e.g., genomic target DNA sequence) or if the binding region is substantially complementary to a single-stranded target nucleic acid sequence (e.g. RNA or an RNA viral genome).

A "genome" is the total genetic constituents of an organism. In the case of eukaryotic organisms, the genome is contained in a haploid set of chromosomes of a cell. In the case of prokaryotic organisms, the genome is contained in a single chromosome, and in some cases one or more extra-chromosomal genetic elements, such as episomes (e.g., plasmids). A viral genome can take the form of one or more single or double stranded DNA or RNA molecules depending on the particular virus.

The term "hapten" refers to a molecule, typically a small molecule that can combine specifically with an antibody, but typically is substantially incapable of being immunogenic except in combination with a carrier molecule.

The term "isolated" in reference to a biological component (such as a nucleic acid molecule, protein, or cell), refers to a biological component that has been substantially separated or purified away from other biological components in the cell of the organism, or the organism itself, in which the component naturally occurs, such as other chromosomal and extra-chromosomal DNA and RNA, proteins, cells, and organelles. Nucleic acid molecules that have been "isolated" include nucleic acid molecules purified by standard purification methods. The term also encompasses nucleic acids prepared by amplification or cloning as well as chemically synthesized nucleic acids.

A "label" is a detectable compound or composition that is conjugated directly or indirectly to another molecule to facilitate detection of that molecule. Specific, non-limiting examples of labels include fluorescent and fluorogenic moieties, chromogenic moieties, haptens, affinity tags, and radioactive isotopes. The label can be directly detectable (e.g., optically detectable) or indirectly detectable (for example, via interaction with one or more additional molecules that are in turn detectable). Exemplary labels in the context of the probes disclosed herein are described below. Methods for labeling nucleic acids, and guidance in the choice of labels useful for various purposes, are discussed, e.g., in Sambrook and Russell, in Molecular Cloning: A Laboratory Manual, 3rd Ed., Cold Spring Harbor Laboratory Press (2001) and Ausubel et al., in Current Protocols in Molecular Biology, Greene Publishing Associates and Wiley-Intersciences (1987, and including updates).

The term "multiplex" refers to embodiments that allow multiple targets in a sample to be detected substantially simultaneously, or sequentially, as desired, using plural different conjugates. Multiplexing can include identifying and/or quantifying nucleic acids generally, DNA, RNA, peptides, proteins, both individually and in any and all combinations. Multiplexing also can include detecting two or more of a gene, a messenger and a protein in a cell in its anatomic context.

A "nucleic acid" is a deoxyribonucleotide or ribonucleotide polymer in either single or double stranded form, and unless otherwise limited, encompasses analogues of natural nucleotides that hybridize to nucleic acids in a manner similar to naturally occurring nucleotides. The term "nucleotide" includes, but is not limited to, a monomer that includes a base (such as a pyrimidine, purine or synthetic analogs thereof) linked to a sugar (such as ribose, deoxyribose or synthetic analogs thereof), or a base linked to an amino acid, as in a peptide nucleic acid (PNA). A nucleotide is one monomer in a polynucleotide. A nucleotide sequence refers to the sequence of bases in a polynucleotide.

A nucleic acid "segment" is a subportion or subsequence of a target nucleic acid molecule. A nucleic acid segment can be derived hypothetically or actually from a target nucleic acid molecule in a variety of ways. For example, a segment of a target nucleic acid molecule (such as a genomic target nucleic acid molecule) can be obtained by digestion with one or more restriction enzymes to produce a nucleic acid segment that is a restriction fragment. Nucleic acid segments can also be produced from a target nucleic acid molecule by amplification, by hybridization (for example, subtractive hybridization), by artificial synthesis, or by any other procedure that produces one or more nucleic acids that correspond in sequence to a target nucleic acid molecule. A particular example of a nucleic acid segment is a binding region.

A "probe" or a "nucleic acid probe" is a nucleic acid molecule that is capable of hybridizing with a target nucleic acid molecule (e.g., genomic target nucleic acid molecule) and, when hybridized to the target, is capable of being detected either directly or indirectly. Thus probes permit the detection, and in some examples quantification, of a target nucleic acid molecule. In particular examples a probe includes a plurality of nucleic acid molecules, which include binding regions derived from the target nucleic acid molecule and are thus capable of specifically hybridizing to at least a portion of the target nucleic acid molecule. A probe can be referred to as a "labeled nucleic acid probe," indicating that the probe is coupled directly or indirectly to a detectable moiety or "label," which renders the probe detectable.

The term "quantum dot" refers to a nanoscale particle that exhibits size-dependent electronic and optical properties due to quantum confinement. Quantum dots have, for example, been constructed of semiconductor materials (e.g., cadmium selenide and lead sulfide) and from crystallites (grown via molecular beam epitaxy), etc. A variety of quantum dots having various surface chemistries and fluorescence characteristics are commercially available from Invitrogen Corporation, Eugene, Oreg. (see, for example, U.S. Pat. Nos. 6,815,064, 6,682596 and 6,649,138. Quantum dots are also commercially available from Evident Technologies (Troy, N.Y.). Other quantum dots include alloy quantum dots such as ZnSSe, ZnSeTe, ZnSTe, CdSSe, CdSeTe, ScSTe, HgSSe, HgSeTe, HgSTe, ZnCdS, ZnCdSe, ZnCdTe, ZnHgS, ZnHgSe, ZnHgTe, CdHgS, CdHgSe, CdHgTe, ZnCdSSe, ZnHgSSe, ZnCdSeTe, ZnHgSeTe, CdHgSSe, CdHgSeTe, InGaAs, GaAlAs, and InGaN quantum dots (Alloy quantum dots and methods for making the same are disclosed, for example, in US Application Publication No. 2005/0012182 and PCT Publication WO 2005/001889).

A "sample" is a biological specimen containing genomic DNA, RNA (including mRNA), protein, or combinations thereof, obtained from a subject. Examples include, but are not limited to, chromosomal preparations, peripheral blood, urine, saliva, tissue biopsy, surgical specimen, bone marrow, amniocentesis samples and autopsy material. In one example, a sample includes genomic DNA or RNA. In some examples, the sample is a cytogenetic preparation, for example which can be placed on microscope slides. In particular examples, samples are used directly, or can be manipulated prior to use, for example, by fixing (e.g., using formalin).

The term "signal generating moiety" refers to a composition or molecule that generates a signal that is detectable by an assay.

The term "specific binding moiety" refers to a member of a binding pair. Specific binding pairs are pairs of molecules that are characterized in that they bind each other to the substantial exclusion of binding to other molecules (for example, specific binding pairs can have a binding constant that is at least 10³ M⁻¹ greater, 10⁴ M⁻¹ greater or 10⁵ M⁻¹ greater than a binding constant for either of the two members of the binding pair with other molecules in a biological sample). Particular examples of specific binding moieties include specific binding proteins (for example, antibodies, lectins, avidins such as streptavidins, and protein A), nucleic acids sequences, and protein-nucleic acids. Specific binding moieties can also include the molecules (or portions thereof) that are specifically bound by such specific binding proteins.

The term "specific binding agent" refers to a molecule that comprises a specific binding moiety conjugated to a signal generating moiety.

A "subject" includes any multi-cellular vertebrate organism, such as human and non-human mammals (e.g., veterinary subjects).

A "target nucleic acid sequence or molecule" is a defined region or particular sequence of a nucleic acid molecule, for example a genome (such as a gene or a region of mammalian genomic DNA containing a gene of interest) or an RNA sequence. In an example where the target nucleic acid sequence is a target genomic sequence, such a target can be defined by its position on a chromosome (e.g., in a normal cell), for example, according to cytogenetic nomenclature by reference to a particular location on a chromosome; by reference to its location on a genetic map; by reference to a hypothetical or assembled contig; by its specific sequence or function; by its gene or protein name, or by any other means that uniquely identifies it from among other genetic sequences of a genome. In some examples, the target nucleic acid sequence is mammalian or viral genomic sequence. In other examples, the target nucleic acid sequence is an RNA sequence.

A "cellular target nucleic acid sequence" is a target nucleic acid sequence (e.g., genomic DNA sequence or RNA sequence) that is present in or extracted from a prokaryotic cell, eukaryotic cell, tissue, virus, or other biological entity. Target nucleic acid sequences may also be present within a probe sequence (e.g., a primary probe sequence that has a portion that binds to a cellular target nucleic acid sequence) or other nucleic acid sequences that are synthesized for use in assays.

In some examples, alterations of a target nucleic acid sequence (e.g., genomic nucleic acid sequence) are "associated with" a disease or condition. That is, detection of the target nucleic acid sequence can be used to infer the status of a sample with respect to the disease or condition. For example, the target nucleic acid sequence can exist in two (or more) distinguishable forms, such that a first form correlates with absence of a disease or condition and a second (or different) form correlates with the presence of the disease or condition. The two different forms can be qualitatively distinguishable, such as by polynucleotide polymorphisms, and/or the two different forms can be quantitatively distinguishable, such as by the number of copies of the target nucleic acid sequence that are present in a cell.

A "vector" is any nucleic acid that acts as a carrier for other ("foreign") nucleic acid sequences that are not native to the vector. When introduced into an appropriate host cell a vector may replicate itself (and, thereby, the foreign nucleic acid sequence) or express at least a portion of the foreign nucleic acid sequence. In one context, a vector is a linear or circular nucleic acid into which a target nucleic acid sequence of interest is introduced (for example, cloned) for the purpose of replication (e.g., production) and/or manipulation using standard recombinant nucleic acid techniques (e.g., restriction digestion). A vector can include nucleic acid sequences that permit it to replicate in a host cell, such as an origin of replication. A vector can also include one or more selectable marker genes and other genetic elements known in the art. Common vectors include, for example, plasmids, cosmids, phage, phagemids, artificial chromosomes (e.g., BAC, PAC, HAC, YAC) and hybrids that incorporate features of more than one of these types of vectors. Typically, a vector includes one or more unique restriction sites (and in some cases a multi-cloning site) to facilitate insertion of a target nucleic acid sequence.

### DETAILED DESCRIPTION OF THE INVENTION

Disclosed herein are and probe systems for use in detection of a target biomolecule in a biological sample containing biomolecules such as proteins, nucleic acids, lipids, hormones, etc. In preferred embodiments, the probes and probe systems are used to detect target nucleic acids such as DNA and RNA in a biological sample. In further preferred embodiments, the probes and probe systems are utilized for in situ hybridization procedures, for example, fluorescence in situ hybridization (FISH), colorimetric in situ hybridization (CISH), and silver in situ hybridization (SISH). In some embodiments, the biological sample includes a tissue section (such as obtained by biopsy) or a cytology sample (such as a Pap smear or blood smear). Other types of assays in which the disclosed conjugates can be used are readily apparent to those skilled in the art, and particular examples are discussed below.

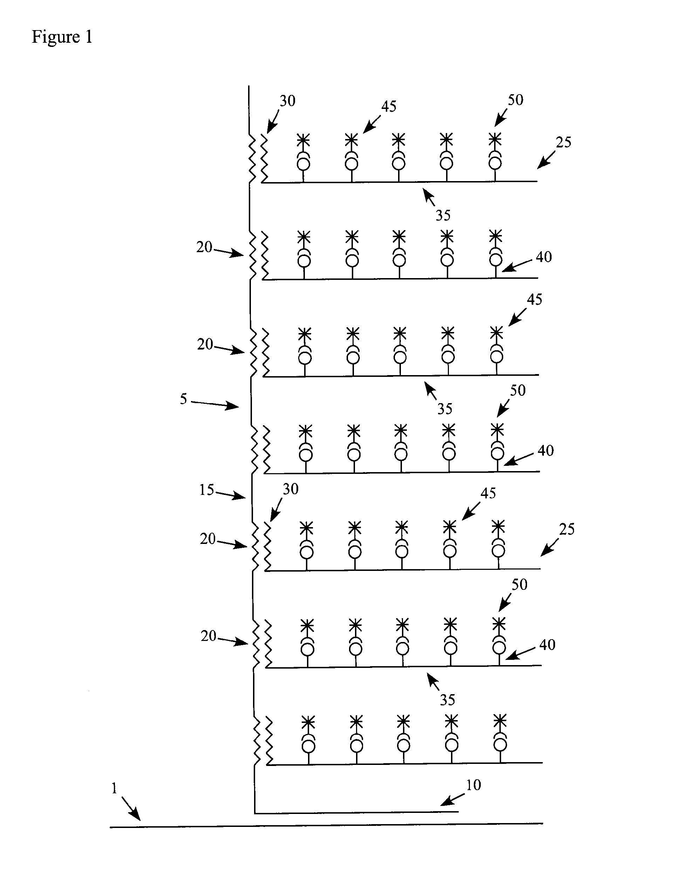

One non-limiting embodiment of the present invention is described in Figure 1. Referring to Figure 1, a sample contains a target nucleic acid **1.** A target nucleic acid probe **5** of the present invention comprises a target probe portion **10** and a detection sequence portion **15** comprising a plurality of detection target sequences **20.** The target probe portion **10** of the target nucleic acid probe 5 is complementary to a region of the target nucleic acid **1** and hybridizes to target nucleic acid **1** under appropriate conditions. The detection target sequences **20** preferably have the same sequence so that the detection sequence portion **15** comprises a plurality of repeat sequences which are the detection target sequences **20.** In some embodiments, the system further comprises detection probes **25** comprising a detection probe portion **30** and detectable moiety portion **35.** The detection probe portion **30** preferably comprises a nucleic acid sequence that is complementary to the detection target sequences **20** of the target nucleic acid probe 5 so that the detection probe portion **30** hybridizes to the detection target sequences **20** under appropriate conditions. The detectable moiety portion **35** preferably comprises a plurality of detectable moieties **40.** For example, in the embodiment depicted, the detectable moieties **40** are preferably haptens. Still referring to Figure 1, in some embodiments, the systems of the present invention further comprise a specific binding agent **45** that binds to the detectable moieties **40.** In some embodiments, the specific binding agent **45** comprises a signal generating moiety **50** that produces a detectable signal, for example, the signal generating moiety may preferably be a fluorescent compound or quantum dot.

The probes and probe systems of the present invention are described in more detail below.

### A. Polytag Probe System

Disclosed herein are probes and probe systems for detection of nucleic acids, and in particular probes and probe systems comprising target nucleic acid probes which comprise a plurality of detection target sequences and detection nucleic acid probes which hybridize to the detection target sequences of the target nucleic acid probes and which further comprise a plurality of detectable moieties, such as haptens. The probes and probe systems disclosed herein can be used to detect a target nucleic acid sequence, such as a genomic target nucleic acid sequence associated with disease or associated with a pathogen, or an RNA target nucleic acid sequence. For example, the probes can be used in *in situ* hybridization procedures that include hybridization of the probes to chromosome preparations, such as metaphase or interphase nuclei or tissue sections.

### 1. Target nucleic acid probes

Disclosed here in is a target nucleic acid probe. In some embodiments, the target nucleic acid probe is a nucleic acid molecule comprising a target probe portion and a detection target portion.

In some embodiments, the target probe portion comprises a nucleic acid sequence that is complementary to a target nucleic acid sequence (e.g., a cellular target nucleic acid sequence or an artificial nucleic acid sequence such as a probe.). In some embodiments, the target probe portion hybridizes to a target nucleic acid sequence under conditions suitable for hybridization, such as conditions suitable for *in situ* hybridization, Southern blotting, or Northern blotting. Preferably, the target probe portion comprises any suitable nucleic acid, such as RNA (Ribonucleic acid), DNA (Deoxyribonucleic acid), LNA (Locked Nucleic Acid), PNA (Peptide Nucleic Acid) or combinations thereof, and can comprise both standard nucleotides such as ribonucleotides and deoxyribonucleotides and nucleotide analogs. In some embodiments, the target probe portion of the target nucleic acid probe is complementary to a cellular target nucleic acid sequence. In these embodiments, the target nucleic acid probe is hybridized directly to the cellular target nucleic acid sequence. In other embodiments, the target probe portion of the target nucleic acid probe is complementary to an artificial target nucleic acid sequence, such as a primary probe. In these embodiments, a primary probe comprising a cellular target probe portion that is complementary to a cellular target nucleic acid sequence and an adaptor portion is hybridized to a cellular target nucleic acid sequence. The target nucleic acid probe preferably comprises a target probe portion (the primary probe target probe portion) that is complementary to and can hybridize with the adaptor portion of the primary probe. Thus, the primary probe is hybridized to the cellular target nucleic acid sequence and then the target nucleic acid probe is hybridized to the primary probe. These embodiments allow the flexible design of probe systems for detection of a desired target nucleic acid sequence. In embodiments where a primary probe is utilized, the adaptor portion of the primary probe allows use of a set of standard target nucleic acid probes that are specific for distinct adaptor portions. The primary probes are synthesized with a portion specific for a cellular target nucleic acid sequence and an adaptor portion that is specific for a particular standardized target nucleic acid probe. This system allows multiplexing using primary probes with distinct adaptor portions and a set of target nucleic acid probes that are specific for each of the distinct adaptor portions.

In some embodiments, the target probe portion is greater than 80% complementary to the target nucleic acid sequence, preferably greater than 90% complementary to the target nucleic acid sequence, more preferably greater than 99% complementary to the nucleic acid sequence, and most preferably about 100% complementary to the nucleic acid sequence. The length of the target probe potion can vary. In some embodiments, the target probe portion comprises a sequence that is complementary to the target nucleic acid of about 10, 20, 50, 100 or 200 nucleotides in length. In some embodiments, the target probe portion that is complementary to the target nucleic acid is up to about 20, 50, 100, 200, 1000, or 5000 nucleotides in length. In some embodiments, the target probe portion that is complementary to the target nucleic acid is from about 10 to about 500, about 10 to about 200 or about 10 to about 100 nucleotides in length. In general, design of these target probe portion is accomplished using practices that are standard in the art. For example, sequences that have self complementarity, such that the resulting probes would either fold upon themselves, or hybridize to each other at the expense of binding to the target nucleic acid, are generally avoided.

One consideration in choosing a length for the target probe portion is the complexity of the sample containing the target nucleic acid. For example, the human genome is approximately 3X10⁹ base pairs in length. Any 10-nucleotide sequence will appear with a frequency of approximately 2,861 times in 3 billion base pairs. A target probe portion of this length would have a poor chance of binding uniquely to a 10 nucleotide region within a target having a sequence the size of the human genome. If the target sequence were within a 3 kb plasmid, however, such an oligonucleotide might have a very reasonable chance of binding uniquely. By this same calculation it can be seen that an oligonucleotide of 16 nucleotides (i.e., a 16-mer) is the minimum length of a sequence that is mathematically likely to appear once in 3X10⁹ base pairs. This level of specificity may also be provided by two or more shorter nucleic acid sequences if they are configured to bind in a cooperative fashion (i.e., such that they can produce the intended complex only if both or all are bound to their intended target sequences), wherein the combination of the short sequences provides the desired specificity.

A second consideration in choosing target probe portion length is the temperature range in which the target probe portion will be expected to function. A 16-mer of average base content (50% G-C bases) will have a calculated Tₘ of about 41°C, depending on, among other things, the concentration of the probe and its target, the salt content of the reaction and the precise order of the nucleotides. As a practical matter, longer target probe portions are usually chosen to enhance the specificity of hybridization. For example, target probe portions of from 20 to 25 nucleotides in length can be used, as they are highly likely to be specific if used in reactions conducted at temperatures which are near their Tₘs (within about 5°C of the Tₘ).

In preferred embodiments, the target probe portion of the target nucleic acid probe is designed taking these considerations into account, so that the target probe portion will hybridize to a target nucleic acid under suitable conditions defined by the user.

The target probe portion can be selected manually, or with the assistance of a computer implemented algorithm that optimizes primer selection based on desired parameters, such as temperature, length, GC content, etc. Numerous computer implemented algorithms or programs for use via the internet or on a personal computer are available. For example, to generate multiple binding regions from a target nucleic acid sequence (e.g., genomic target nucleic acid sequence), regions of sequence devoid of repetitive (or other undesirable, e.g., background-producing) nucleic acid sequence are identified, for example manually or by using a computer algorithm. Within a target nucleic acid sequence (e.g., genomic target nucleic acid sequence) that spans several to several-hundred kilobases, typically numerous binding regions that are substantially or completely free of repetitive (or other undesirable, e.g., background-producing) nucleic acid sequences are identified.

In some embodiments, the target nucleic acid probe further comprises a detection target portion either 5' or 3' to the target probe portion, preferably 3' to the target probe portion. In some preferred embodiments, the detection target portion of the target nucleic acid probe comprises one or more detection target sequences. In some embodiments, the detection target sequence is a sequence that is complementary to a detection probe nucleic acid sequence (described in more detail below) so that the detection target sequence can be detected by hybridization with a detection probe.

In some embodiments, the detection portion comprises a plurality of detection target sequences. In some embodiments, the detection target sequences in a given target nucleic acid probe are identical or substantially identical so that the detection target sequences can hybridize with the same detection probe. In other embodiments, the sequences of the detection target sequences are varied so that the detection target sequences hybridize to two or more different detection probes. It will be understood that the present invention provides support for ranges falling with the following specified ranges. The number of detection target sequences included within the detection portion can vary. Accordingly, in some embodiments, the detection portion comprises greater than about 5, 10, 20, 30, 50, or 100 detection target sequences, up to about 100 detection target sequences. In some embodiments, the detection portion comprises between about 5 to 30, 5 to 50, 10 to 50, 10 to 100, 10 to 200, 20 to 40, 20 to 50, 20 to 100 or 20 to 200 detection target sequences. The length of the detection portion can vary. In some embodiments, the overall length of the detection portion is from about 20 to about 2000, about 100 to about 2000, about 20 to about 500, about 100 to about 2000, about 100 to about 1500, about 100 to about 1000, about 200 to about 2000, about 200 to about 1500, about 200 to about 1000, or about 200 to about 500 nucleotides in length. The length of the detection target sequences can vary. In some embodiments, the detection target sequences are greater than about 10, 20, 50, or 75 nucleotides up to about 100 or 200 nucleotides in length. In some embodiments, the detection target sequences are separated by spacer sequences. In some embodiments, the spacer sequences range from about 10 nucleotides in length up to about 20, 50 or 100 nucleotides in length. In some embodiments, the spacer sequences comprise one or more restriction sites for a restriction endonuclease.

The detection target sequences are using the considerations such as those described for design of the target probe portion. In some embodiments, the detection target sequences are designed so as to efficiently and/or specifically hybridize with a detection probe. In some embodiments, the base composition of the detection target sequences (and corresponding detection probe sequences) is selected such that hybridization of the target nucleic acid probe to the target nucleic acid sequence and the hybridization of the detection probe to the detection target sequences occur under substantially the same conditions, for example, temperature, time, buffer and salt concentrations.

The target nucleic acid probes can be synthesized by any known method. In some embodiments, the sequences encoding the target nucleic acid probes are cloned into a plasmid expression vector. The target nucleic probe is preferably transcribed from the vector with an RNA polymerase to provide an RNA molecule encoding the target nucleic acid probe. In some embodiments, the target nucleic acid probe is chemically synthesized, for example, using phosphoramidite analogs. In some embodiments, DNA probes are synthesized by propagation, purification and restriction digestion of plasmid DNA to provide a DNA molecule encoding the target nucleic acid probe. The double stranded DNA can be subsequently melted into single strands for use in hybridization protocols. In some embodiments, the target nucleic acid probes are synthesized by asymmetric PCR. In some embodiments, one primer, could for example, be a nucleic acid analog ( e.g., LNA). This process generates a probe with the target specific portion containing locked nucleotides and the detection target portion being made from standard dNTP's. In some embodiments, the LNA containing primer contains a biotin to facilitate purification of the desired strand.

### 2. Detection Probes and Specific Binding Agents

Disclosed herein is a detection probe. In some embodiments, the detection probe is a nucleic acid molecule comprising a detection probe portion and a detectable moiety portion. In some embodiments, the detectable moiety portion of the detection probe comprises a plurality of detectable moieties that are detectable with a specific binding agent.

In some embodiments, the detection probe portion comprises a nucleic acid sequence that is complementary to a detection target sequence as described above. In some embodiments, the nucleic acid sequence of the detection probe portion hybridizes to a detection target sequence under conditions suitable for hybridization, such as conditions suitable for *in situ* hybridization, Southern blotting, or Northern blotting. Preferably, the detection probe portion comprises any suitable nucleic acid, such as RNA, DNA, LNA, PNA or combinations thereof, and can comprise both standard nucleotides such as ribonucleotides and deoxyribonucleotides, as well as nucleotide analogs. LNA and PNA are two examples of nucleic acid analogs that form hybridization complexes that are more stable (i.e., have an increased Tₘ) than those formed between DNA and DNA or DNA and RNA. LNA and PNA analogs can be combined with traditional DNA and RNA nucleosides during chemical synthesis to provide hybrid nucleic acid molecules than can be used as probes. Use of the LNA and PNA analogs allows modification of hybridization parameters such as the Tₘ of the hybridization complex. This allows the design of detection probes that hybridize to the detection target sequences of the target nucleic acid probes under conditions that are the same or similar to the conditions required for hybridization of the target probe portion to the target nucleic acid sequence.

The length of the detection probe portion can vary, but is designed for complementarity to a detection target sequence of a target nucleic acid probe. In some embodiments, the detection target sequences are greater than about 10, 20, 50, or 75 nucleotides up to about 100 or 200 nucleotides in length. The detection probe portion is preferably designed using the considerations such as those described for design of the target probe portion. In some embodiments, the detection probe portion is designed so as to efficiently and/or specifically hybridize with a detection target sequence. In some embodiments, the base composition of the detection probe portion (and corresponding detection target sequences) is selected such that hybridization of the target nucleic acid probe to the target nucleic acid sequence and the hybridization of the detection probe to the detection target sequences occur under substantially the same conditions, for example, temperature, time, buffer and salt concentrations.

In some embodiments the detectable moiety portion comprises one or more detectable moieties. In some embodiments, the detectable moieties are directly detectable, while in other embodiments, the detectable moieties are indirectly detectable. In some embodiments, the detectable moieties are incorporated into the detection probe. In some embodiments, the detectable moieties are signal generating moieties that produce a detectable signal. In some embodiments, the detectable moiety is conjugated to nucleotides or nucleotide analogs used in the synthesis of the detection probe. For example, nucleoside phosphoramidites that are conjugated to a desired detectable moiety are used to synthesize a detection probe via chemical synthesis as is known in the art. In some embodiments, the detectable moiety portion comprises a plurality of detectable moieties. For example, in some embodiments, the detectable moiety portion comprises from about 5 to about 50, about 5 to about 25, about 5 to about 20, about 5 to about 15 or from about 5 to about 10 detectable moieties. It will be recognized that the combination of multiple detection target sequences on the target nucleic acid probe allows hybridization of multiple detection probes to each target nucleic acid probe. When each detection probe comprises a plurality of detectable moieties, amplification of the detection signal occurs.

In some embodiments, the detectable moiety is detected indirectly. In some embodiments, the detectable moiety is a first member of a binding molecule pair that includes first and second members. In these embodiments, nucleotides conjugated to a first member of a binding pair are incorporated into the detection probe, preferably via the use nucleoside phosphoramidites conjugated to the first member of the binding pair. A specific binding agent comprising the second member of the binding pair (i.e., a specific binding moiety) conjugated to a signal generating moiety is then used detect the detection probe via binding to the first member of the binding pair. Examples of suitable binding molecules pairs include, but are not limited to, avidin and biotin and hapten and anti-hapten antibodies. For example, in some embodiments, the detectable moiety portion of the detection probe comprises a plurality of biotinylated nucleotides. These biotinylated nucleotides are detected by the use of compounds comprising avidin conjugated to a directly detectable moiety. In other embodiments, the detectable moiety portion of the detection probe comprises a plurality of haptenylated nucleotides. These haptenylated nucleotides are detected by the use of compounds comprising anti-hapten antibodies conjugated to a directly detectable moiety.

Accordingly, in some embodiments, disclosed herein are detection probes that comprise one or more nucleotides that are conjugated to the first member of a binding molecule pair. In some embodiments, the first member of the binding molecule pair is a hapten. In some embodiments, the detectable moiety portion of the detection probe is a nucleic acid molecule that incorporates dNTPs covalently attached to hapten molecules (such as a nitro-aromatic compound (e.g., dinitrophenyl (DNP)), biotin, fluorescein, digoxigenin, etc.). Methods for conjugating haptens and other labels to dNTPs (e.g., to facilitate incorporation into labeled probes) are well known in the art. For examples of procedures, see, e.g., U.S. Pat. Nos. 5,258,507, 4,772,691, 5,328,824, and 4,711,955. Indeed, numerous labeled dNTPs are available commercially, for example from Invitrogen Detection Technologies (Molecular Probes, Eugene, Oreg.). A label can be directly or indirectly attached of a dNTP at any location on the dNTP, such as a phosphate (e.g., α, β or γ phosphate) or a sugar.

A variety of haptens may used in the detectable moiety portion of the detection probe. Such haptens include, but are not limited to, pyrazoles, particularly nitropyrazoles; nitrophenyl compounds; benzofurazans; triterpenes; ureas and thioureas, particularly phenyl ureas, and even more particularly phenyl thioureas; rotenone and rotenone derivatives, also referred to herein as rotenoids; oxazole and thiazoles, particularly oxazole and thiazole sulfonamides; coumarin and coumarin derivatives; cyclolignans, exemplified by Podophyllotoxin and Podophyllotoxin derivatives; and combinations thereof. Specific examples of haptens include, but are not limited to, 2,4-Dintropheyl (DNP), Biotin, Fluorescein deratives (FITC, TAMRA, Texas Red, etc.), Digoxygenin (DIG), 5-Nitro-3-pyrozolecarbamide (nitropyrazole, NP), 4,5,-Dimethoxy-2-nitrocinnamide (nitrocinnamide, NCA), 2-(3,4-Dimethoxyphenyl)-quinoline-4-carbamide (phenylquinolone, DPQ), 2,1,3-Benzoxadiazole-5-carbamide (benzofurazan, BF), 3-Hydroxy-2-quinoxalinecarbamide (hydroxyquinoxaline, HQ), 4-(Dimethylamino)azobenzene-4'-sulfonamide (DABSYL), Rotenone isoxazoline (Rot), (E)-2-(2-(2-oxo-2,3-dihydro-1H-benzo[b][1,4]diazepin-4-yl)phenozy)acetamide (benzodiazepine, BD), 7-(diethylamino)-2-oxo-2H-chromene-3-carboxylic acid (coumarin 343, CDO), 2-Acetamido-4-methyl-5-thiazolesulfonamide (thiazolesulfonamide, TS), and p-Mehtoxyphenylpyrazopodophyllamide (Podo). These haptens and their use in probes are described in more detail in co-owned applications US Pat. Publ. Nos. 20080305497, 20080268462, and 20080057513.

In embodiments where the detectable moiety portion of the detection probe comprises haptens, the second member of the binding molecule pair is preferably a molecule that binds to the hapten such as an antigen binding molecule. Examples of suitable antigen binding molecules include, but are not limited to, antibodies, immunoglobulins or immunoglobulin-like molecules (including by way of example and without limitation, IgA, IgD, IgE, IgG and IgM), antibody fragments such as F(ab')₂ fragments, Fab' fragments, Fab'-SH fragments and Fab fragments as are known in the art, recombinant antibody fragments (such as sFv fragments, dsFv fragments, bispecific sFv fragments, bispecific dsFv fragments, F(ab)'₂ fragments, single chain Fv proteins ("scFv"), disulfide stabilized Fv proteins ("dsFv"), diabodies, and triabodies (as are known in the art), and camelid antibodies (see, for example, U.S. Pat. Nos. 6,015,695; 6,005,079-5,874,541; 5,840,526; 5,800,988; and 5,759,808). In preferred embodiments, a detectable moiety that generates a detectable signal is attached, covalently or otherwise, to the antigen binding molecule. Examples of suitable second binding pair members include, but are not limited to anti-DNP, anti-biotin, anti-FITC, anti-DIG, anti-NP, anti-NCA, anti-DPQ, anti-BF, anti-HQ, anti-DABSYL, anti-Rot, anti-BD, anti-CDO, anti-TS, and anti-Podo antibodies that are conjugated to a detectable moiety that generates a detectable signal. In further embodiments, second member of the binding molecule pair is an anti-hapten primary antibody that does not comprise a detectable moiety. In these embodiments, a secondary anti-antibody (such as a goat anti-mouse IgG antibody) that comprises a detectable moiety that generates a signal is utilized for generating a detectable signal.

As described above, the detection probe can be directly detectable or indirectly detectable. In some direct detection embodiments, the detection probe comprises detectable moieties (e.g., signal generating moieties) that generate a detectable signal, while in some indirect detection embodiments, a specific binding agent comprising a member of a binding molecule pair (such as a secondary antibody) that is conjugated to a signal generating moiety that generates a detectable signal is utilized. In these embodiments, a variety of signal generating moieties that generate a detectable signal may be incorporated into the detection probe or conjugated to the member of the binding pair.

In preferred embodiments, the signal generating moiety can be detected by any known or yet to be a discovered mechanism including absorption, emission and/or scattering of a photon (including radio frequency, microwave frequency, infrared frequency, visible frequency and ultra-violet frequency photons). Signal-generating moieties include colored, fluorescent, phosphorescent and luminescent molecules and materials, catalysts (such as enzymes) that convert one substance into another substance to provide a detectable difference (such as by converting a colorless substance into a colored substance or vice versa, or by producing a precipitate or increasing sample turbidity), and paramagnetic and magnetic molecules or materials.

Particular examples of signal-generating moieties include fluorescent molecules (or fluorochromes). Numerous fluorochromes are known to those of skill in the art, and can be selected, for example from Invitrogen, e.g., see, The Handbook--A Guide to Fluorescent Probes and Labeling Technologies, Invitrogen Detection Technologies, Molecular Probes, Eugene, Oreg.). Examples of particular fluorophores that can be attached (for example, chemically conjugated) to a nucleic acid molecule or protein such as an antigen binding molecule include, but are not limited to, 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcouluarin (Coumaran 151); cyanosine; 4',6-diaminidino-2-phenylindole (DAPI); 5',5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate (FITC), and QFITC (XRITC); 2',7'-difluorofluorescein (OREGON GREEN^{™}); fluorescamine; IR144; IR1446; Malachite Green isothiocyanate; 4-methylumbelliferone; ortho cresolphthalein; nitrotyrosine; pararosaniline; Phenol Red; B-phycoerythrin; ophthaldialdehyde; pyrene and derivatives such as pyrene, pyrene butyrate and succinimidyl 1-pyrene butyrate; Reactive Red 4 (Cibacron^{™} Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives.

Other suitable fluorophores include thiol-reactive europium chelates which emit at approximately 617 nm (Heyduk and Heyduk, Analyt. Biochem. 248:216-27, 1997; J. Biol. Chem. 274:3315-22, 1999), as well as GFP, Lissamine.TM., diethylaminocoumarin, fluorescein chlorotriazinyl, naphthofluorescein, 4,7-dichlororhodamine and xanthene (as described in U.S. Pat. No. 5,800,996 to Lee et al.) and derivatives thereof. Other fluorophores known to those skilled in the art can also be used, for example those available from Invitrogen Detection Technologies, Molecular Probes (Eugene, Oreg.) and including the ALEXA FLUOR^{™} series of dyes (for example, as described in U.S. Pat. Nos. 5,696,157, 6,130,101 and 6,716,979), the BODIPY series of dyes (dipyrrometheneboron difluoride dyes, for example as described in U.S. Pat. Nos. 4,774,339, 5,187,288, 5,248,782, 5,274,113, 5,338,854, 5,451,663 and 5,433,896), Cascade Blue (an amine reactive derivative of the sulfonated pyrene described in U.S. Pat. No. 5,132,432) and Marina Blue (U.S. Pat. No. 5,830,912).

In addition to the fluorochromes described above, a fluorescent label can be a fluorescent nanoparticle, such as a semiconductor nanocrystal, e.g., a QUANTUM DOT^{™} (obtained, for example, from QuantumDot Corp, Invitrogen Nanocrystal Technologies, Eugene, Oreg.; see also, U.S. Pat. Nos. 6,815,064, 6,682,596 and 6,649,138). Semiconductor nanocrystals are microscopic particles having size-dependent optical and/or electrical properties. When semiconductor nanocrystals are illuminated with a primary energy source, a secondary emission of energy occurs of a frequency that corresponds to the bandgap of the semiconductor material used in the semiconductor nanocrystal. This emission can be detected as colored light of a specific wavelength or fluorescence. Semiconductor nanocrystals with different spectral characteristics are described in e.g., U.S. Pat. No. 6,602,671. Semiconductor nanocrystals that can be coupled to a variety of biological molecules (including dNTPs and/or nucleic acids) or substrates by techniques described in, for example, Bruchez et. al. (1998) Science 281:2013-6, Chan et al. (1998) Science 281:2016-8, and U.S. Pat. No. 6,274,323.

Formation of semiconductor nanocrystals of various compositions are disclosed in, e.g., U.S. Pat. Nos. 6,927,069; 6,914,256; 6,855,202; 6,709,929; 6,689,338; 6,500,622; 6,306,736; 6,225,198; 6,207,392; 6,114,038; 6,048,616; 5,990,479; 5,690,807; 5,571,018; 5,505,928; 5,262,357 and in U.S. Patent Publication No. 2003/0165951 as well as PCT Publication No. 99/26299 (published May 27, 1999). Separate populations of semiconductor nanocrystals can be produced that are identifiable based on their different spectral characteristics. For example, semiconductor nanocrystals can be produced that emit light of different colors based on their composition, size or size and composition. For example, quantum dots that emit light at different wavelengths based on size (565 nm, 655 nm, 705 nm, or 800 nm emission wavelengths), which are suitable as fluorescent labels in the probes disclosed herein are available from Invitrogen.

Additional signal-generating moieties include, for example, radioisotopes (such as ³H, ³⁵S and ³²P), metal chelates such as DOTA and DPTA chelates of radioactive or paramagnetic metal ions like Gd³⁺, and liposomes.

Signal-generating moieties also include enzymes, for example horseradish peroxidase, alkaline phosphatase, acid phosphatase, glucose oxidase, β-galactosidase, β-glucuronidase or β-lactamase. Where the detectable label includes an enzyme, a chromogen, fluorogenic compound, or luminogenic compound can be used in combination with the enzyme to generate a detectable signal (numerous of such compounds are commercially available, for example, from Invitrogen Corporation, Eugene Oreg.). Particular examples of chromogenic compounds include diaminobenzidine (DAB), 4-nitrophenylphospate (pNPP), fast red, bromochloroindolyl phosphate (BCIP), nitro blue tetrazolium (NBT), BCIP/NBT, fast red, AP Orange, AP blue, tetramethylbenzidine (TMB), 2,2'-azino-di-[3-ethylbenzothiazoline sulphonate] (ABTS), o-dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-.beta.-D-galactopyranoside (MU-Gal), p-nitrophenyl-α-D-galactopyranoside (PNP), 5-bromo-4-chloro-3-indolyl-.beta.-D-glucuronide (X-Gluc), 3-amino-9-ethyl carbazol (AEC), fuchsin, iodonitrotetrazolium (INT), tetrazolium blue and tetrazolium violet.

Alternatively, an enzyme can be used in a metallographic detection scheme. For example, SISH procedures involve metallographic detection schemes for identification and localization of a hybridized genomic target nucleic acid sequence. Metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate. (See, for example, U.S. Patent Application Publication No. 2005/0100976, PCT Publication No. 2005/003777 and U.S. Patent Application Publication No. 2004/0265922). Metallographic detection methods include using an oxido-reductase enzyme (such as horseradish peroxidase) along with a water soluble metal ion, an oxidizing agent and a reducing agent, again to for form a detectable precipitate. (See, for example, U.S. Pat. No. 6,670,113).

In some embodiments, the signal-generating moiety is a fluorescent protein. Fluorescent proteins also can be used as a carrier, or can be coupled to a carrier, to facilitate visualization. For example, green fluorescent protein (GFP) was originally isolated from the light-emitting organ of the jellyfish *Aequorea victoria*. Chimeric GFP fusions can be expressed in situ by gene transfer into cells, and can be localized to particular sites within the cell by appropriate targeting signals. Spectral variants with blue, cyan and yellowish-green emissions have been successfully generated from the Aequorea GFP, but none exhibit emission maxima longer than 529 nm. GFP-like proteins have been isolated from Anthozoa (coral animals) that significantly expanded the range of colors available for biological applications. The family of 'GFP-like proteins' deposited in sequence databases now includes approximately 30 significantly different members. Fluorescent proteins refers to proteins that can become spontaneously fluorescent through the autocatalytic synthesis of a chromophore. Proteins that fluoresce at red or far-red wavelengths (red fluorescent proteins or RFPs) are known. RFPs can be used in combination with other fluorescent proteins that fluoresce at shorter wavelengths for both multicolor labeling and fluorescence resonance energy transfer (FRET) experiments. Commercially available RFPs are derived from two wild-type GFP-like proteins. DsRed (drFP583) has excitation and emission maxima at 558 nm and 583 nm, respectively. A far-red fluorescent protein was generated by mutagenesis of a chromoprotein that absorbs at 571 nm. HcRed1 (Clontech) has excitation and emission maxima at 588 nm and 618 nm, respectively. The fluorescent protein that emits fluorescence at the longest wavelength (without any mutations being introduced) is eqFP611, cloned from the sea anemone *Entacmaea quadricolor.* This protein absorbs at 559 nm and emits at 611 nm.

The detection probes can be synthesized by any suitable, known nucleic acid synthesis method. In some embodiments, the detection probes are chemically synthesized using phosphoramidite nucleosides and/or phosphoramidite nucleoside analogs. For example, in some embodiments, the detection probes are synthesized by using standard RNA or DNA phosphoramidite nucleosides. In some embodiments, the detection probes are synthesized using either LNA phosphoramidites or PNA phosphoramidites, alone or in combination with standard phosphoramidite nucleosides. In some embodiments, the detectable moieties are incorporated into the detection probe during chemical synthesis. For example, in some embodiments, detectable moieties, such as haptens, are introduced on basic phosphoramidites containing the desired detectable moieties. Other methods can also be used for detection probe synthesis. For example, a primer made from LNA analogs or a combination of LNA analogs and standard nucleotides can be used for transcription of the remainder of the probe. As another example, a primer comprising detectable moieties is utilized for transcription of the rest of the probe. In still other embodiments, segments of the probe produced, for example, by transcription or chemical synthesis, may be joined by enzymatic or chemical ligation.

### B. Use of Probes and Probe Systems

The present invention provides methods of using the disclosed probes and probes systems. For example, the probes can be used to detect a target nucleic acid molecule. In one example, the method includes contacting one or more of the disclosed target nucleic acid probes with a sample that includes nucleic acid molecules under conditions sufficient to permit hybridization between the nucleic acid molecules in the sample and the target nucleic acid probes. The sample is then contacted with the detection probe under conditions sufficient to permit hybridization between the detection probe and the target nucleic acid probes. The detection probe is then detected, for example by contacting the sample with a compound comprising a binding partner of a compound incorporated into the detection probe, or by assaying the detection probe directly.

The probes and probe systems of the present disclosure be used for nucleic acid detection, such as *in situ* hybridization procedures (e.g., fluorescence *in situ* hybridization (FISH), chromogenic *in situ* hybridization (CISH) and silver *in situ* hybridization (SISH)). Hybridization between complementary nucleic acid molecules is mediated via hydrogen bonding, which includes Watson-Crick, Hoogsteen or reversed Hoogsteen hydrogen bonding between complementary nucleotide units. For example, adenine and thymine are complementary nucleobases that pair through formation of hydrogen bonds. If a nucleotide unit at a certain position of a probe of the present disclosure is capable of hydrogen bonding with a nucleotide unit at the same position of a DNA or RNA molecule (e.g., a target nucleic acid sequence) then the oligonucleotides are complementary to each other at that position. The probe and the DNA or RNA are complementary to each other when a sufficient number of corresponding positions in each molecule are occupied by nucleotide units which can hydrogen bond with each other, and thus produce detectable binding. A probe need not be 100% complementary to its target nucleic acid sequence (e.g., genomic target nucleic acid sequence) to be specifically hybridizable. However sufficient complementarity is needed so that the probe binds, duplexes, or hybridizes only or substantially only to a target nucleic acid sequence when that sequence is present in a complex mixture (e.g., total cellular DNA or RNA).

*In situ* hybridization involves contacting a sample containing a target nucleic acid sequence (e.g., genomic target nucleic acid sequence) in the context of a metaphase or interphase chromosome preparation (such as a cell or tissue sample mounted on a slide) with a probe (i.e., the target nucleic acid probe described above) specifically hybridizable or specific for the target nucleic acid sequence (e.g., genomic target nucleic acid sequence). The slides are optionally pretreated, e.g., to remove paraffin or other materials that can interfere with uniform hybridization. The chromosome sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess target nucleic acid probe, and detection of specific labeling of the chromosome target is performed. According to some embodiments of the present invention, the detection is facilitated by hybridization of a detection probe to the target nucleic acid probe. The detection probe may be detected by direct detection or by indirect detection.

For example, in some direct detection embodiments, the detection probe is labelled with one or more fluorescent compounds, and the sample is analyzed by fluorescence microscopy or imaging. In some indirect detection embodiments, the detection probe comprises a plurality of detectable moieties comprising first members of a binding pair (i.e., a hapten or biotin) which are detected by contacting the sample with a compound comprising a second member of the binding pair (i.e., anti-hapten antibody or avidin) conjugated to a detectable moiety (i.e., a fluorochrome or quantum dot). For a general description of in situ hybridization procedures, see, e.g., U.S. Pat. No. 4,888,278. Numerous procedures for fluorescence in situ hybridization (FISH), chromogenic in situ hybridization (CISH) and silver in situ hybridization (SISH) are known in the art. For example, procedures for performing FISH are described in U.S. Pat. Nos. 5,447,841, 5,472,842, 5,427,932, and for example, in Pinkel et al., Proc. Natl. Acad. Sci. 83:2934-2938, 1986; Pinkel et al., Proc. Natl. Acad. Sci. 85:9138-9142, 1988, and Lichter et al., Proc. Natl. Acad. Sci. 85:9664-9668, 1988. CISH is described in, e.g., Tanner et al., Am. J. Pathol. 157:1467-1472, 2000, and U.S. Pat. No. 6,942,970. Additional detection methods are provided in U.S. Pat. No. 6,280,929. Exemplary procedures for detecting viruses by in situ hybridization can be found in Poddighe et al., J. Clin. Pathol. 49:M340-M344, 1996.

Numerous reagents and detection schemes can be employed in conjunction with FISH, CISH, and SISH procedures to improve sensitivity, resolution, or other desirable properties. As discussed above, detection probes labeled with fluorophores (including fluorescent dyes and QUANTUM DOTS^{™}) can be directly optically detected when performing FISH. Alternatively, the detection probe can be labeled with a non-fluorescent molecule, such as a hapten (such as the following non-limiting examples: biotin, digoxygenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Detection probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., QUANTUM DOT^{™}) or with another indirectly detectable moiety, or can be contacted with one or more additional specific binding agents (e.g., secondary or specific antibodies), which can in turn be labeled with a fluorophore. Optionally, the detectable label is attached directly to the antibody, receptor (or other specific binding agent). Alternatively, the detectable label is attached to the binding agent via a linker, such as a hydrazide thiol linker, a polyethylene glycol linker, or any other flexible attachment moiety with comparable reactivities. For example, a specific binding agent, such as an antibody, a receptor (or other anti-ligand), avidin, or the like can be covalently modified with a fluorophore (or other label) via a heterobifunctional polyalkylene glycol linker such as a heterobifunctional polyethylene glycol (PEG) linker. A heterobifunctional linker combines two different reactive groups selected, e.g., from a carbonyl-reactive group, an amine-reactive group, a thiol-reactive group and a photo-reactive group, the first of which attaches to the label and the second of which attaches to the specific binding agent.

In other examples, the detection probe, or specific binding agent (such as an antibody, e.g., a primary antibody, receptor or other binding agent) comprises an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publication Nos. 2006/0246524; 2006/0246523, and U.S. patent application publication number 2007011715

It will be appreciated by those of skill in the art that by appropriately selecting labeled detection probes and/or labeled binding pairs, multiplex detection schemes can be produced to facilitate detection of multiple target nucleic acid sequences (e.g., genomic target nucleic acid sequences) in a single assay (e.g., on a single cell or tissue sample or on more than one cell or tissue sample). For example, a first detection probe that corresponds to a first target nucleic acid probe can be labeled with a first hapten, such as biotin, while a second detection probe that corresponds to a second target nucleic acid sequence can be labeled with a second hapten, such as DNP. Following exposure of the sample to the probe sets, the bound probes can be detected by contacting the sample with a first specific binding agent (in this case avidin labeled with a first fluorophore, for example, a first spectrally distinct QUANTUM DOT^{™}, e.g., that emits at 585 nm) and a second specific binding agent (in this case an anti-DNP antibody, or antibody fragment, labeled with a second fluorophore (for example, a second spectrally distinct QUANTUM DOT^{™}, e.g., that emits at 705 nm). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Standard fluorescence microscopes are an inexpensive tool for the detection of reagents and probes incorporating fluorescent compounds, such as quantum dot bioconjugates. Since quantum dot conjugates are virtually photo-stable, time can be taken with the microscope to find regions of interest and adequately focus on the samples. Quantum dot conjugates are useful any time bright photo-stable emission is required and are particularly useful in multicolor applications where only one excitation source/filter is available and minimal crosstalk among the colors is required.

### C. Targets

A target nucleic acid molecule can be any selected nucleic acid, such as DNA or RNA. In particular embodiments, the target sequence is a genomic target sequence or genomic subsequence, for example from a eukaryotic genome, such as a human genome. In some embodiments, the target nucleic acid is cytoplasmic RNA. In some embodiments, the target nucleic acid molecule is selected from a pathogen, such as a virus, bacteria, or intracellular parasite, such as from a viral genome. In some embodiments, the target nucleic acid sequence is a genomic sequence, such as eukaryotic (e.g., mammalian) or viral genomic sequence. Target nucleic acid probes can be generated which correspond to essentially any genomic target sequence that includes at least a portion of unique non-repetitive DNA. For example, the genomic target sequence can be a portion of a eukaryotic genome, such as a mammalian (e.g., human), fungal or intracellular parasite genome. Alternatively, a genomic target sequence can be a viral or prokaryotic genome (such as a bacterial genome), or portion thereof. In a specific example, the genomic target sequence is associated with an infectious organism (e.g., virus, bacteria, fungi).

In some embodiments, the target nucleic acid molecule can be a sequence associated with (e.g., correlated with, causally implicated in, etc.) a disease. In some embodiments, a target sequence is selected that is associated with a disease or condition, such that detection of hybridization can be used to infer information (such as diagnostic or prognostic information for the subject from whom the sample is obtained) relating to the disease or condition. In certain embodiments, the selected target nucleic acid molecule is a target nucleic acid molecule associated with a neoplastic disease (or cancer). In some embodiments, the genomic target sequence can include at least one at least one gene associated with cancer (e.g., HER2, c-Myc, n-Myc, Ab1, Bc12, Bc16, R1, p53, EGFR, TOP2A, MET, or genes encoding other receptors and/or signaling molecules, etc.) or chromosomal region associated with a cancer. In some embodiments, the target nucleic acid sequence can be associated with a chromosomal structural abnormality, e.g., a translocation, deletion, or reduplication (e.g., gene amplification or polysomy) that has been correlated with a cancer. In some embodiments, the target nucleic acid sequence encompasses a genomic sequence that is reduplicated or deleted in at least some neoplastic cells. The target nucleic acid sequence can vary substantially in size, such as at least 20 base pairs in length, at least 100 base pairs in length, at least 1000 base pairs in length, at least 50,000, at least 100,000, or even at least 250,000 base pairs in overall length.

The target nucleic acid sequence (e.g., genomic target nucleic acid sequence) can span any number of base pairs. In some embodiments, the target nucleic acid sequence spans at least 1000 base pairs. In specific examples, a target nucleic acid sequence (e.g., genomic target nucleic acid sequence) is at least 10,000, at least 50,000, at least 100,000, at least 150,000, at least 250,000, or at least 500,000 base pairs in length (such as 100 kb to 600 kb, 200 kb to 500 kb, or 300 kb to 500 kb). In examples, where the target nucleic acid sequence is from a eukaryotic genome (such as a mammalian genome, e.g., a human genome), the target sequence typically represents a small portion of the genome (or a small portion of a single chromosome) of the organism (for example, less than 20%, less than 10%, less than 5%, less than 2%, or less than 1% of the genomic DNA (or a single chromosome) of the organism). In some examples where the target sequence (e.g., genomic target nucleic acid sequence) is from an infectious organism (such as a virus), the target sequence can represent a larger proportion (for example, 50% or more) or even all of the genome of the infectious organism.

In specific non-limiting examples, a target nucleic acid sequence (e.g., genomic target nucleic acid sequence) associated with a neoplasm (for example, a cancer) is selected. Numerous chromosome abnormalities (including translocations and other rearrangements, reduplication or deletion) have been identified in neoplastic cells, especially in cancer cells, such as B cell and T cell leukemias, lymphomas, breast cancer, colon cancer, neurological cancers and the like. Therefore, in some examples, at least a portion of the target nucleic acid sequence (e.g., genomic target nucleic acid sequence) is reduplicated or deleted in at least a subset of cells in a sample.

Translocations involving oncogenes are known for several human malignancies. For example, chromosomal rearrangements involving the SYT gene located in the breakpoint region of chromosome 18q11.2 are common among synovial sarcoma soft tissue tumors. The t(18q11.2) translocation can be identified, for example, using probes with different labels: the first probe includes nucleic acid molecules generated from a target nucleic acid sequence that extends distally from the SYT gene, and the second probe includes nucleic acid generated from a target nucleic acid sequence that extends 3' or proximal to the SYT gene. When probes corresponding to these target nucleic acid sequences (e.g., genomic target nucleic acid sequences) are used in an in situ hybridization procedure, normal cells, which lacks a t(18q11.2) in the SYT gene region, exhibit two fusion (generated by the two labels in close proximity) signals, reflecting the two intact copies of SYT. Abnormal cells with a t(18q11.2) exhibit a single fusion signal.

Numerous examples of reduplication of genes involved in neoplastic transformation have been observed, and can be detected cytogenetically by in situ hybridization using the disclosed probes. In one example, the target nucleic acid sequence (e.g., genomic target nucleic acid sequence) is selected include a gene (e.g., an oncogene) that is reduplicated in one or more malignancies (e.g., a human malignancy). For example, HER2, also known as c-erbB2 or HER2/neu, is a gene that plays a role in the regulation of cell growth (a representative human HER2 genomic sequence is provided at GENBANK^{™} Accession No. NC_000017, nucleotides 35097919-35138441). The gene codes for a 185 kd transmembrane cell surface receptor that is a member of the tyrosine kinase family. HER2 is amplified in human breast, ovarian, and other cancers. Therefore, a HER2 gene (or a region of chromosome 17 that includes the HER2 gene) can be used as a genomic target nucleic acid sequence to generate probes that include nucleic acid molecules with binding regions specific for HER2.

In other examples, a target nucleic acid sequence (e.g., genomic target nucleic acid sequence) is selected that is a tumor suppressor gene that is deleted (lost) in malignant cells. For example, the p16 region (including D9S1749, D9S1747, p16(INK4A), p14(ARF), D9S1748, p15(INK4B), and D9S 1752) located on chromosome 9p21 is deleted in certain bladder cancers. Chromosomal deletions involving the distal region of the short arm of chromosome 1 (that encompasses, for example, SHGC57243, TP73, EGFL3, ABL2, ANGPTL1, and SHGC-1322), and the pericentromeric region (e.g., 19p13-19q13) of chromosome 19 (that encompasses, for example, MAN2B1, ZNF443, ZNF44, CRX, GLTSCR2, and GLTSCR1)) are characteristic molecular features of certain types of solid tumors of the central nervous system.

The aforementioned examples are provided solely for purpose of illustration and are not intended to be limiting. Numerous other cytogenetic abnormalities that correlate with neoplastic transformation and/or growth are known to those of skill in the art. Target nucleic acid sequences (e.g., genomic target nucleic acid sequences), which have been correlated with neoplastic transformation and which are useful in the disclosed methods and for which disclosed probes can be prepared, also include the EGFR gene (7p12; e.g., GENBANK^{™} Accession No. NC_000007, nucleotides 55054219-55242525), the C-MYC gene (8q24.21; e.g., GENBANK^{™} Accession No. NC_000008, nucleotides 128817498-128822856), D5S271 (5p15.2), lipoprotein lipase (LPL) gene (8p22; e.g., GENBANK^{™} Accession No. NC_000008, nucleotides 19841058-19869049), RB1 (13q14; e.g., GENBANK^{™} Accession No. NC_000013, nucleotides 47775912-47954023), p53 (17p13.1; e.g., GENBANK^{™} Accession No. NC_000017, complement, nucleotides 7512464-7531642)), N-MYC (2p24; e.g., GENBANK^{™} Accession No. NC_000002, complement, nucleotides 151835231-151854620), CHOP (12q13; e.g., GENBANK^{™} Accession No. NC_000012, complement, nucleotides 56196638-56200567), FUS (16p11.2; e.g., GENBANK^{™} Accession No. NC_000016, nucleotides 31098954-31110601), FKHR (13p14; e.g., GENBANK^{™} Accession No. NC_000013, complement, nucleotides 40027817-40138734), as well as, for example: ALK (2p23; e.g., GENBANK^{™} Accession No. NC_000002, complement, nucleotides 29269144-29997936), Ig heavy chain, CCND1 (11q13; e.g., GENBANK^{™} Accession No. NC_000011, nucleotides 69165054 . .. 69178423), BCL2 (18q21.3; e.g., GENBANK^{™} Accession No. NC_000018, complement, nucleotides 58941559-59137593), BCL6 (3q27; e.g., GENBANK^{™} Accession No. NC_000003, complement, nucleotides 188921859-188946169), MALF1, API (1p32-p31; e.g., GENBANK^{™} AccessionNo. NC_000001, complement, nucleotides 59019051-59022373), TOP2A (17q21-q22; e.g., GENBANK^{™} Accession No. NC_000017, complement, nucleotides 35798321-35827695), TMPRSS (21q22.3; e.g., GENBANK^{™} Accession No. NC_000021, complement, nucleotides 41758351-41801948), ERG (21q22.3; e.g., GENBANK^{™} Accession No. NC_000021, complement, nucleotides 38675671-38955488); ETV1 (7p21.3; e.g., GENBANK^{™} Accession No. NC_000007, complement, nucleotides 13897379-13995289), EWS (22q12.2; e.g., GENBANK^{™} Accession No. NC_000022, nucleotides 27994271-28026505); FLI1 (11q24.1-q24.3; e.g., GENBANK^{™} Accession No. NC_000011, nucleotides 128069199-128187521), PAX3 (2q35-q37; e.g., GENBANK^{™} AccessionNo. NC_000002, complement, nucleotides 222772851-222871944), PAX7 (1p36.2-p36.12; e.g., GENBANK^{™} Accession No. NC_000001, nucleotides 18830087-18935219, PTEN (10q23.3; e.g., GENBANK^{™} Accession No. NC_000010, nucleotides 89613175-89716382), AKT2 (19q13.1-q13.2; e.g., GENBANK^{™} Accession No. NC_000019, complement, nucleotides 45431556-45483036), MYCL1 (1p34.2; e.g., GENBANK^{™} Accession No. NC_000001, complement, nucleotides 40133685-40140274), REL (2p13-p12; e.g., GENBANK^{™} Accession No. NC_000002, nucleotides 60962256-61003682) and CSF1R (5q33-q35; e.g., GENBANK^{™} Accession No. NC_000005, complement, nucleotides 149413051-149473128). A disclosed target nucleic acid probe or method may include a region of the respective human chromosome containing at least any one (or more, as applicable) of the foregoing genes. For example, the target nucleic acid sequence for some disclosed probes or methods includes any one of the foregoing genes and sufficient additional contiguous genomic sequence (whether 5' of the gene, 3' of the gene, or a combination thereof) for a total of at least 100,000 base pairs (such as at least 250,000, or at least 500,000 base pairs) or a total of between 100,000 and 500,000 base pairs.

In certain embodiments, the probe specific for the target nucleic acid molecule is assayed (in the same or a different but analogous sample) in combination with a second probe that provides an indication of chromosome number, such as a chromosome specific (e.g., centromere) probe. For example, a probe specific for a region of chromosome 17 containing at least the HER2 gene (a HER2 probe) can be used in combination with a CEP 17 probe that hybridizes to the alpha satellite DNA located at the centromere of chromosome 17 (17p11.1-q11.1). Inclusion of the CEP 17 probe allows for the relative copy number of the HER2 gene to be determined. For example, normal samples will have a HER2/CEP 17 ratio of less than 2, whereas samples in which the HER2 gene is reduplicated will have a HER2/CEP17 ratio of greater than 2.0. Similarly, CEP centromere probes corresponding to the location of any other selected genomic target sequence can also be used in combination with a probe for a unique target on the same (or a different) chromosome.

In other examples, a target nucleic acid sequence (e.g., genomic target nucleic acid sequence) is selected from a virus or other microorganism associated with a disease or condition. Detection of the virus- or microorganism-derived target nucleic acid sequence (e.g., genomic target nucleic acid sequence) in a cell or tissue sample is indicative of the presence of the organism. For example, the probe can be selected from the genome of an oncogenic or pathogenic virus, a bacterium or an intracellular parasite (such as Plasmodium falciparum and other Plasmodium species, Leishmania (sp.), Cryptosporidium parvum, Entamoeba histolytica, and Giardia lamblia, as well as Toxoplasma, Eimeria, Theileria, and Babesia species).

In some examples, the target nucleic acid sequence (e.g., genomic target nucleic acid sequence) is a viral genome. Exemplary viruses and corresponding genomic sequences (GENBANK^{™} RefSeq Accession No. in parentheses) include human adenovirus A (NC_001460), human adenovirus B (NC_004001), human adenovirus C(NC_001405), human adenovirus D (NC_002067), human adenovirus E (NC_003266), human adenovirus F (NC_001454), human astrovirus (NC_001943), human BK polyomavirus (V01109; GI:60851) human bocavirus (NC_007455), human coronavirus 229E (NC_002645), human coronavirus HKU1 (NC_006577), human coronavirus NL63 (NC_005831), human coronavirus OC43 (NC_005147), human enterovirus A (NC_001612), human enterovirus B (NC_001472), human enterovirus C(NC_001428), human enterovirus D (NC_001430), human erythrovirus V9 (NC_004295), human foamy virus (NC_001736), human herpesvirus 1 (Herpes simplex virus type 1) (NC_001806), human herpesvirus 2 (Herpes simplex virus type 2) (NC_001798), human herpesvirus 3 (Varicella zoster virus) (NC_001348), human herpesvirus 4 type 1 (Epstein-Barr virus type 1) (NC_007605), human herpesvirus 4 type 2 (Epstein-Barr virus type 2) (NC_009334), human herpesvirus 5 strain AD 169 (NC_001347), human herpesvirus 5 strain Merlin Strain (NC_006273), human herpesvirus 6A (NC_001664), human herpesvirus 6B (NC_000898), human herpesvirus 7 (NC_001716), human herpesvirus 8 type M (NC_003409), human herpesvirus 8 type P (NC_009333), human immunodeficiency virus 1 (NC_001802), human immunodeficiency virus 2 (NC_001722), human metapneumovirus (NC_004148), human papillomavirus-1 (NC_001356), human papillomavirus-18 (NC. _001357), human papillomavirus-2 (NC_001352), human papillomavirus-54 (NC_001676), human papillomavirus-61 (NC_001694), human papillomavirus-cand90 (NC_004104), human papillomavirus RTRX7 (NC_004761), human papillomavirus type 10 (NC_001576), human papillomavirus type 101 (NC_008189), human papillomavirus type 103 (NC_008188), human papillomavirus type 107 (NC_009239), human papillomavirus type 16 (NC_001526), human papillomavirus type 24 (NC_001683), human papillomavirus type 26 (NC_001583), human papillomavirus type 32 (NC_001586), human papillomavirus type 34 (NC_001587), human papillomavirus type 4 (NC_001457), human papillomavirus type 41 (NC_001354), human papillomavirus type 48 (NC_001690), human papillomavirus type 49 (NC_001591), human papillomavirus type 5 (NC_001531), human papillomavirus type 50 (NC_001691), human papillomavirus type 53 (NC_001593), human papillomavirus type 60 (NC_001693), human papillomavirus type 63 (NC_001458), human papillomavirus type 6b (NC_001355), human papillomavirus type 7 (NC_001595), human papillomavirus type 71 (NC_002644), human papillomavirus type 9 (NC_001596), human papillomavirus type 92 (NC_004500), human papillomavirus type 96 (NC_005134), human parainfluenza virus 1 (NC_003461), human parainfluenza virus 2 (NC_003443), human parainfluenza virus 3 (NC_001796), human parechovirus (NC_001897), human parvovirus 4 (NC_007018), human parvovirus B19 (NC_000883), human respiratory syncytial virus (NC_001781), human rhinovirus A (NC_001617), human rhinovirus B (NC_001490), human spumaretrovirus (NC_001795), human T-lymphotropic virus 1 (NC_001436), human T-lymphotropic virus 2 (NC_001488).

In certain examples, the target nucleic acid sequence (e.g., genomic target nucleic acid sequence) is from an oncogenic virus, such as Epstein-Barr Virus (EBV) or a Human Papilloma Virus (HPV, e.g., HPV16, HPV18). In other examples, the target nucleic acid sequence (e.g., genomic target nucleic acid sequence) is from a pathogenic virus, such as a Respiratory Syncytial Virus, a Hepatitis Virus (e.g., Hepatitis C Virus), a Coronavirus (e.g., SARS virus), an Adenovirus, a Polyomavirus, a Cytomegalovirus (CMV), or a Herpes Simplex Virus (HSV).

### D. Kits

Disclosed are kits including at least one target nucleic acid probe disclosed herein, and optionally, at least one primary probe. For example, kits for in situ hybridization procedures such as FISH, CISH, and/or SISH include at least one target nucleic acid probe as described herein, and optionally, at least one primary probe. In some embodiments, the kits further include one or more detection probes for use in conjunction with the at least one target nucleic acid probes. In some embodiments, the kits further include at least specific binding agent for use in conjunction with the one or more detection probes. Accordingly, kits can include one or more target nucleic acid probes, one or more detection probes, and one or more specific binding agents.

The kits can also include one or more reagents for performing an in situ hybridization assay, or for producing a probe. For example, a kit can include at least one nucleic acid molecule (or population of such molecules), along with one or more buffers, labeled dNTPs, a labeling enzyme (such as a polymerase), primers, nuclease free water, and instructions for producing a labeled probe.

In one example, the kit includes one or more target nucleic acid probes, one or more detection probes and one or more specific binding agents along with buffers and other reagents for performing in situ hybridization such as paraffin pretreatment buffer, protease(s) and protease buffer, prehybridization buffer, hybridization buffer, wash buffer, counterstain(s), mounting medium, or combinations thereof. The kit can optionally further include control slides for assessing hybridization and signal of the probe.

### E. Automation

A person of ordinary skill in the art will appreciate that embodiments of the method disclosed herein for using hapten conjugates can be automated. Ventana Medical Systems, Inc. is the assignee of a number of United States patents disclosing systems and methods for performing automated analyses, including U.S. Pat. Nos. 5,650,327, 5,654,200, 6,296,809, 6,352,861, 6,827,901 and 6,943,029, and U.S. published application Nos. 20030211630 and 20040052685. Particular embodiments of polymeric hapten staining procedures can be conducted using various automated processes.

Additional details concerning exemplary working embodiments are provided in the working examples.

### EXAMPLES

### EXAMPLE 1 DETECTION OF mRNA IN FIXED TISSUE USING POLYTAG PROBES

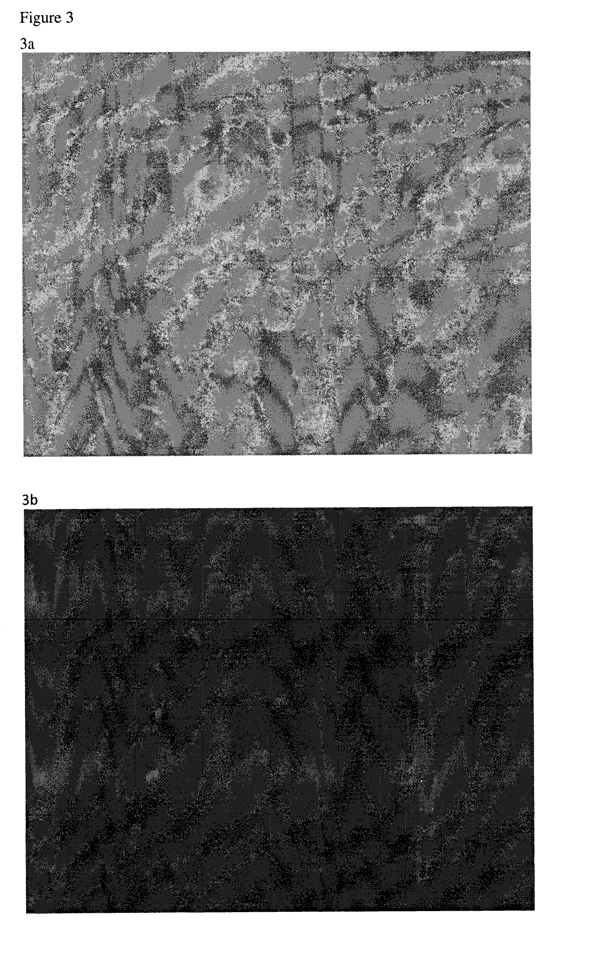

Formalin fixed paraffin embedded xenograft tissue was deparaffinized using xylene and conditioned for hybridization by sequential treatments with formalin, acid denaturation (0.3 M HCl), sodium citrate/Tween 20 buffer and protease. After conditioning, PolyTag riboprobes (SEQ ID NOs:7-18; 1 ug/ml diluted in a formamide containing hybridization buffer) were deposited on the tissue and denatured at 80°C before six hour hybridizations at 65°C. Unbound and non-specifically bound probe was removed with three high stringency washes (0.1xSSC, 75°C). Hapten-labeled anti-PolyTag detection oligonucleotides (SEQ ID NO:24; 5 ug/ml diluted in formamide containing hybridization buffer) were deposited on the tissue and denatured at 60°C before one hour hybridizations at 37°C. Unbound and non-specifically bound detection oligo was removed using two low stringency washes (2xSSC, 37°C). Haptens bound to mRNA targets were detected using cognate mouse anti-hapten monoclonal antibodies conjugated to quantum dot (Invitrogen) diluted in buffered diluent containing casein (20 nM). Nuclei were counterstained using DAPI. The slides were dehydrated using increasing ethanol washes and coverslipped using Cytoseal 60 mounting medium. Fluorescent signals were captured using interferometric spectral imaging. See Figure 3.

### EXAMPLE 2 DETECTION OF RNA FIXED TO A GLASS SLIDE BY POLYTAG PROBES

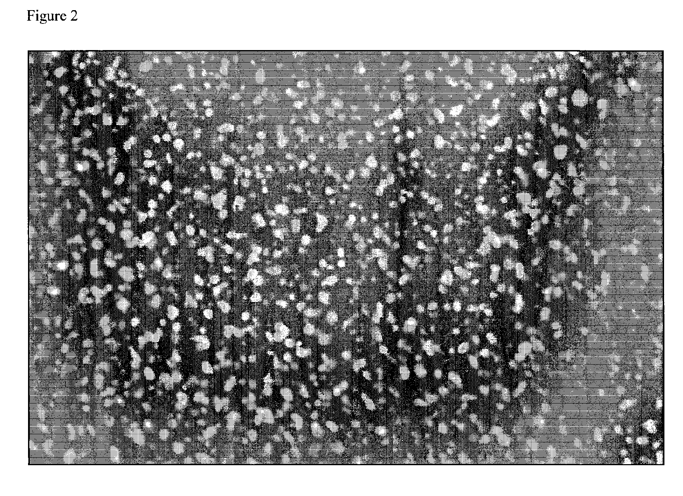

Dot blot staining. One microgram to one nanogram of *in vitro* transcribed RNA target were diluted in Spotting Solution II (Genorama) and 1 ul deposited onto aminosilane coated microarray slides (Genorama) and, following drying, cross-linked using UV light. The slides were blocked using buffered antibody diluent containing casein and PolyTag riboprobes (SEQ ID NOs:19-21; 1 ug/ml diluted in formamide hybridization buffer) deposited on the slides and denatured at 80°C before six hour hybridizations at 65°C. Unbound and non-specifically bound probe was removed with three high stringency washes (0.1xSSC, 75°C). Hapten-labeled anti-PolyTag detection oligonucleotides (SEQ ID NOs: 27, 30 and [add gina SEQ ID NO]; 5 ug/ml diluted in formamide hybridization buffer) were deposited on the tissue and denatured at 60°C before one hour hybridizations at 37°C. Unbound and non-specifically bound detection oligo was removed using two low stringency washes (2xSSC, 37°C). Haptens bound to mRNA targets were detected using cognate mouse anti-hapten monoclonal antibodies conjugated to quantum dot (Invitrogen) diluted in buffered diluent containing casein (20 nM). The slides were dehydrated using increasing ethanol washes and coverslipped using Cytoseal 60 mounting medium. Qdot signals, captured using interferometric spectral imaging, were deconvolved into separate analyte channels and overlayed for visualization using ImagePro software. See Fig. 2.

### EXAMPLE 3 DETECTION OF NUCLEAR DNA IN FIXED TISSUE BY POLYTAG PROBES

A chromosome 17 centromere-specific polytag probe (SEQ ID NO:6) was generated by transcription of a linearized plasmid with T7 RNA polymerase. Formalin fixed paraffin embedded tissue in 5uM slices on a glass microscope slide was subjected to hybridization and detection steps as described below.After deparaffinization as above, slides were treated with a citrate/Tween 20 buffer solution at 90degrees centigrade for 12 minutes and a protease treatment at 37 degrees for 8 minutes. Polytag probe formulated in a formamide containing buffer was applied to the slide which was then heated to 92 degrees for 8 minutes to denature the double stranded DNA target. After hybridization for 8 hours the slides were washed twice at 72 degrees for 8 minutes with 2XSSC.

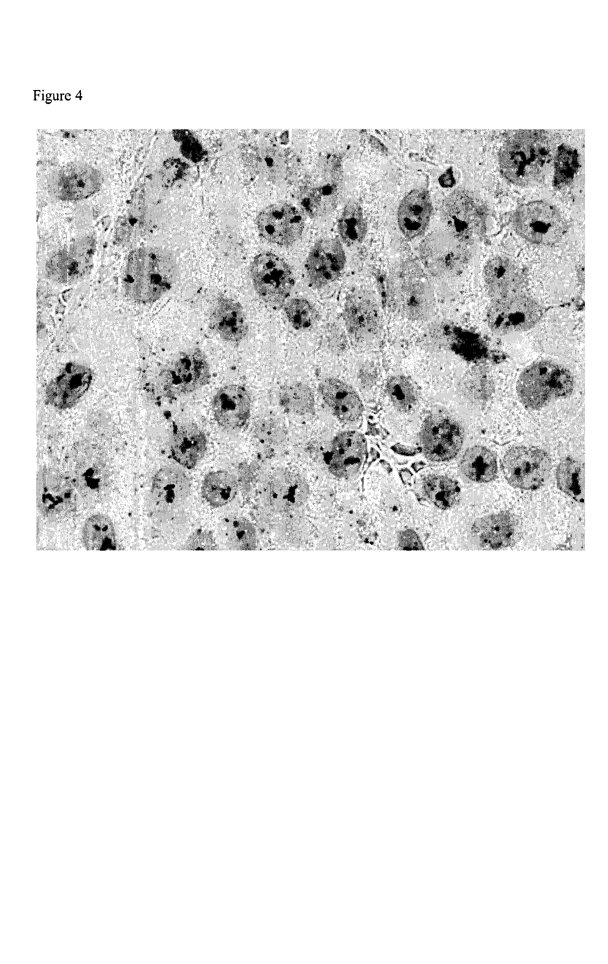

The hapten labeled detection oligo similarly formulated was added (SEQ ID NO:24). After heating to 55 degrees for 8 minutes hybridization was allowed to proceed for 1 hour at 37 degrees. After two washes with 2XSSC at 37 degrees the hybridized probe was detected with a rabbit anti DNP monoclonal antibody followed by washing and application of a Peroxidase conjugated Goat anti - Rabbit antibody. The peroxidase signal was detected by silver deposition. See Fig. 4.

### EXAMPLE 4 TAGRET NUCLEIC ACID AND DETECTION PROBE SETS

Target nucleic acid probes (RNA) were synthesized by transcribing the probe from a DNA template encoding the probe sequence. The following detection target sequences (also called polytags) were arranged in operable association with the target probe sequences (sequences that hybridize to the target DNA) and were repeated from 10 to 40 times in the full-length target nucleic acid probes.
POLYTAG SOPHIA
   SEQ ID NO:1: (CAUCAGCAGGACGCACUGACCACCAUGAAGGUGCUCUUCU)N
POLYTAG RAQUEL:
   SEQ ID NO:2
   (AGAACAAGAAUACUACCGUCAUGCACUUGAUCCGGCACGGUCACUAGU)N
POLYTAG QINGXIA:
   SEQ ID NO:3 (UUACACCUCACCGACAAUAGAAGAUCGUCCUGGCACUGAACUUGCCU) N
POLYTAG EVA:
   SEQ ID NO:4 (UCCGCAGUAACGCUUAAUCGCUCCAGACGACACCCAUGG)N
POLYTAG GINA:
   SEQ ID NO:5
   RNA target nucleic acid probes specific for the chromosome 17 centromere:
SEQ ID NO:6

RNA target nucleic acid probes specific for actin mRNA:
1. SEQ ID NO:7
2. SEQ ID NO: 8
3. SEQ ID NO: 9
4. SEQ ID NO: 10
5. SEQ ID NO: 11
6. SEQ ID NO:12
7. SEQ ID NO:13
8. SEQ ID NO:14
9. SEQ ID NO: 15
10. SEQ ID NO: 16
11. SEQ ID NO: 17
12. SEQ ID NO: 18

Where n = 56; i.e., 56 repeats of the polytag.

RNA target nucleic acid probes specific for human 18s ribosomal RNA:
SEQ ID NO: 19
SEQ ID NO: 20
SEQ ID NO: 21

Where n = 40; i.e., 40 repeats of the polytag.

Detection oligonucleotides were synthesized on a Mermade Oligonucelotide Synthesizer using standard phosphoramidites and protocols provided by the manufacturer. The haptens, indicated by R, were introduced on an abasic phosphoramidite containing the desired hapten. The Sophia detection probe was labeled with DNP (Dinitrophenol) and used with an anti-DNP antibody coupled to Qdots emitting at 585nM. The Raquel detection probe was labeled with TS (Thiazole Sulfonamide) and used with an anti-TS antibody coupled to Qdots emitting at 655nM. The Qingxia detection probe was labeled with DC (Diethyl Coumarin) and used with an anti-DC antibody coupled to Qdots emitting at 605nM. The Eva detection probe was labeled with BF (Benzofurazan) and used with an anti-BZ antibody coupled to Qdots emitting at 525nM.

### Sophie:

SEQ ID NO:22
   Sophie 5:
   RTATTTTRTATTTTRTATTTTRTATTTTRTAGAAGAGCACCTTCATGGTGGTCAGTGCGTCCTGCTGATG
SEQ ID NO:23
   Sophie 10:
SEQ ID NO:24
   Sophie 15:

### Raquel:

SEQ ID NO:25
   Raquel 5:
SEQ ID NO:26
   Raquel 10: Raquel 15:

### QingXia:

SEQ ID NO:28
   QingXia 5:
SEQ ID NO:29
   QingXia 10:
SEQ ID NO: 30
   QingXia 15:
Eva:
   SEQ ID NO:31
Eva 5:
   RTATTTTRTATTTTRTATTTTRTATTTTRCCATGGGTGTCGTCTGGAGCGATTAAGCGTTACTGCGGA
SEQ ID NO:32
   Eva 10:
SEQ ID NO:33
   Eva 15:

### Gina:

SEQ ID NO:34
   Gina 5 :
SEQ ID NO:35
   Gina 10:
SEQ ID NO:36
   Gina

Short polytag sequences suitable for use with LNA or PNA detection probes:
SEQ ID NO:37 R25_810: AGAAGTATCGTTCCGATCTAACGCG
SEQ ID NO:38 R25_998: ACGCTATTACGATTACGACGTGCGA
SEQ ID NO:39 R25_1486: TACGATCGCATCGAGTCGCAGATAT
SEQ ID NO:40 R25_1426: CGCACGCATAGTTAGTCGGATATAC
SEQ ID NO:41 R30_587: CTAGCTCCGATCCGTGATAACGTGC
SEQ ID NO:42 R30_927: ATGTTACGACCGGCGATCTTATACG
SEQ ID NO: 43 R30_1 : (GTACATCCTCCGGTTGCGAATATAGCGAAC)

Sequences suitable for use in hapten labeled LNA detection probes:
SEQ ID NO:44 SSophia 1 CTAGATCTCTCGAGACATGC
SEQ ID NO:45 SSophia 2 TTCTAGATCTCTCGAGACATGCACA

### EXAMPLE 5 DETECTION WITH A SINGLE STRANDED DNA POLYTAG PROBE

### A) Construction of single-stranded DNA PolyTag probe

This example describes application of the PolyTag system to detect nucleic acid target in a cell or tissue. A five-copy direct repeat of a unique sequence (sequence KK5) was chemically synthesized that has no significant sequence homology to the human genome. The PolyTag KK was cloned in the plasmid vector puc 19, and the sequence of the clone verified by sequencing. A ten-copy direct repeat version (KK10) by duplicating KK5 was constructed with standard molecular biology techniques.

The human PTEN gene was selected as the gene of interest, and only intronic sequences were chosen for PCR primer design and amplification. Thirty-six DNA fragments were amplified, all about 200 base pairs long with designed primers (primer sequences and corresponding PCR amplicon sequences are listed below). A phosphate group was added to the 5' end of DNA fragments with T4 polynucleotide kinase, and then the DNA fragments were purified from agarose gel. The PTEN PCR fragments were ligated with KK5 or KK10 sequence linearized with restriction enzyme SmaI with T4 DNA ligase. The single-stranded DNA PTEN PolyTag probe was made by: 1) With the ligation products as template, PCR amplification of PTEN fragments with KK5 or KK10 sequence joined together with PTEN specific forward primer (used previously to generate the PTEN PCR fragment) plus one common 5' phosphorylated primer that is complementary to the KK sequence (KK5Rp); 2) Lambda exonuclease treatment of PCR fragments generated single stranded DNA PolyTag probe for the PTEN, because the enzyme preferably degrades DNA strand with 5' phosphate group.

### B) Examples of Using PolyTag ssDNA probe for detection of nucleic acid target of interest.

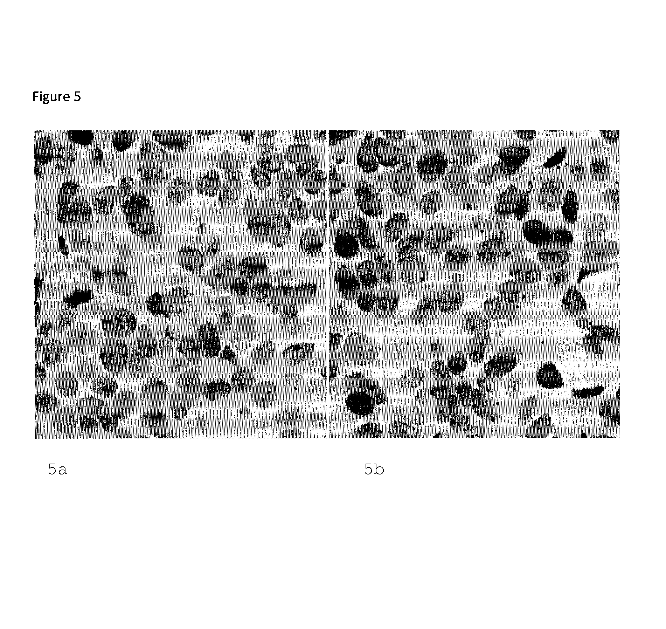

In this example, the PTEN PolyTag ssDNA probe (the probe) was used to detect its target from human cell on paraffin-embedded tissue slides; 30 out of 36 PTEN PCR fragments (number 1-30 in the PTEN PCR sequence list) were used in this example. The tissue was deparaffinied, hydrated, prehybridized. Hybridization was carried out in 45°C for 6 hours and no blocking DNA was used. Post probe hybridization washing with 2xSSC was done at 72°C for 8 minutes and repeating a total of three times. The procedures for KK detection oligo hybridization and washing are: 1) Denature at 55°C for 8 minutes; 2) Hybridization at 45°C for 1 hour; 3) Washing with 2 x SSC three times at 45°C. Detecting signal with SISH was as published. Both five-copy PolyTag and ten-copy PolyTag probes gave clear and strong signals. See Figure 5a for five-copy results and Figure 5b for ten-copy results. The detection procedure was automated on the Ventana Medical Systems BenchMark XT platform, and it should be universally adaptable to manual as well as other semi-automatic or full automatic detection systems.

As a variation of the PolyTag design, one copy of a unique adaptor sequence 30 to 80 nucleotides long was attached to the target nucleic acid (which is different from the PolyTag sequence); the PolyTag amplifier (PolyTag + adaptor sequence region complementary to the adapter sequence) is then hybridized to the target sequence. Specifically, the hybridization occurs between the adaptor sequence on the target nucleic acid probe and the adaptor sequence region on the PolyTag probe. This design was used to detect the PTEN gene on paraffin-embedded human tissue slides. The single stranded PolyTag amplifier was constructed, and adaptor-tagged gene specific single stranded DNA probes with PCR amplification followed by Lambda exonuclease treatment with appropriate template and primers as described in (A).

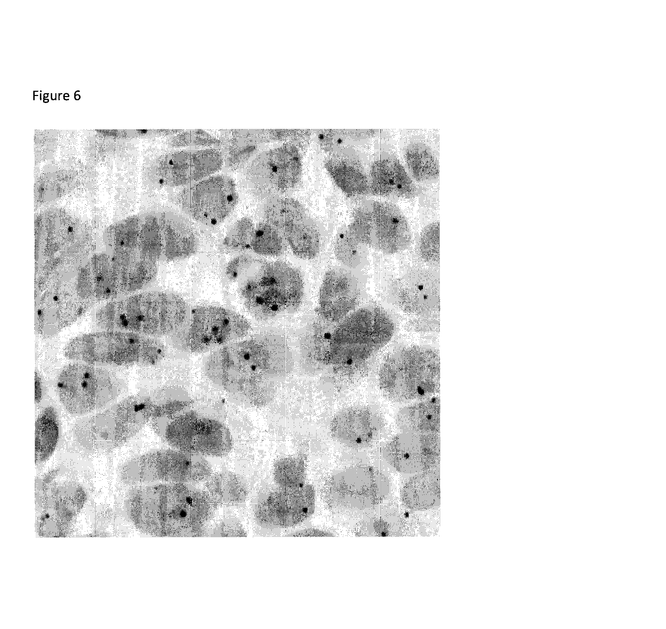

The tissue was deparaffinized, hydrated, and prehybridized. Hybridization was carried out in 45°C for 6 hours and no blocking DNA was used. Post probe hybridization washing with 2xSSC was done at 72°C for 8 minutes and repeating a total of three times. The procedure for PolyTag amplifier hybridization and washing are similar to that to the probe, but with only 1 hour hybridization time. The procedures for KK detection oligo hybridization and washing are: 1) Denature at 55°C for 8 minutes; 2) Hybridization at 45°C for 1 hour; 3) Washing with 2 x SSC three times at 45°C. Detecting signal with SISH was as published. The PolyTag probes (ten-copy) gave clear and strong signals. See Figure 6. The detection procedure was automated on the Ventana Medical Systems BenchMark XT platform, but it should be universally adaptable to manual as well as other semi-automatic or full automatic detection systems.

### C) Nucleic Acid Sequences

PolyTag KK5 sequence (SEQ ID NO:46):
KK Detection Oligo sequence (SEQ ID NO:47): denotes the hapten 2,4-Dinitrophenol (DNP)].
KK5Rp (SEQ ID NO:48) :
   5' [Phos]TGCCTGCAGGTCGACAGGAGCTAG
Adaptor Sequence A (SEQ ID NO:49) :
Human PTEN PCR sequences number 1 to 36:
   >PTEN-PCR01 (SEQ ID NO:50)
   >PTEN-PCR02 (SEQ ID NO:51)
   >PTEN-PCR03 (SEQ ID NO:52)
   >PTEN-PCR04 (SEQ ID NO:53)
   >PTEN-PCR05 (SEQ ID NO:54)
   >PTEN-PCR06 (SEQ ID NO:55)
   >PTEN-PCR07 (SEQ ID NO:56)
   >PTEN-PCR08 (SEQ ID NO:57) >PTEN-PCR09 (SEQ ID NO:58)
   >PTEN-PCR10 (SEQ ID NO:59)
   >PTEN-PCR11 (SEQ ID NO:60)
   >PTEN-PCR12 (SEQ ID NO:61)
   >PTEN-PCR13 (SEQ ID NO:62)
   >PTEN-PCR14 (SEQ ID NO:63)
   >PTEN-PCR15 (SEQ ID NO:64)
   >PTEN-PCR16 (SEQ ID NO:65)
   >PTEN-PCR17 (SEQ ID NO:66)
   >PTEN-PCR18 (SEQ ID NO:67)
   >PTEN-PCR19 (SEQ ID NO:68)
   >PTEN-PCR20 (SEQ ID NO:69)
   >PTEN-PCR21 (SEQ ID NO:70)
   >PTEN-PCR22 (SEQ ID NO:71)
   >PTEN-PCR23 (SEQ ID NO:72)
   >PTEN-PCR24 (SEQ ID NO:73)
   >PTEN-PCR25 (SEQ ID NO:74)
   >PTEN-PCR26 (SEQ ID NO:75)
   >PTEN-PCR27 (SEQ ID NO:76)
   >PTEN-PCR28 (SEQ ID NO:77)
   >PTEN-PCR29 (SEQ ID NO:78)
   >PTEN-PCR30 (SEQ ID NO:79)
   >PTEN-PCR31 (SEQ ID NO:80)
   >PTEN-PCR32 (SEQ ID NO:81)
   >PTEN-PCR33 (SEQ ID NO:82)
   >PTEN-PCR34 (SEQ ID NO:83)
   >PTEN-PCR35 (SEQ ID NO:84)
   >PTEN-PCR36 (SEQ ID NO:85)

## Claims

1. A method for detecting a first cellular target nucleic acid sequence in situ in a tissue sample comprising:
contacting said tissue sample with a first nucleic acid molecule comprising a target probe portion comprising a nucleic acid sequence complementary to said first cellular target nucleic acid sequence and a detection target portion comprising a plurality of first detection target sequences that are complementary to at least one detection probe nucleic acid sequence and non-complementary to said target nucleic acid sequence, under conditions where said target probe portion of said first nucleic acid molecule hybridizes directly to said cellular target nucleic acid sequence;
contacting said first nucleic acid molecule with a plurality of second nucleic acid molecules each comprising a detection probe portion complementary to said detection target sequences of said first nucleic acid molecule and a detectable moiety portion comprising at least one first detectable moiety either 5' or 3' to said detection probe portion, wherein said at least one first detectable moiety is incorporated into the nucleic acid molecule, under conditions such that said detection probe portion of said second nucleic acid molecule hybridizes to said first detection target sequences of said first nucleic acid molecule; and
detecting said at least one first detectable moiety.

2. The method of Claim 1, wherein said target probe portion of said first nucleic acid molecule and said target nucleic acid sequence and said detection probe portion of said second nucleic acid and said detection target sequences of said first nucleic acid molecule have melting points within about 10 degrees Celsius.

3. The method of Claim 1, wherein said cellular target nucleic acid sequence is selected from the group consisting of genomic DNA, nuclear DNA, RNA, mRNA, and cytoplasmic nucleic acid.

4. The method of Claim 1, further comprising contacting the tissue sample with a third nucleic acid molecule comprising a target probe portion comprising a nucleic acid sequence complementary to a second cellular target nucleic acid sequence and a detection target portion comprising a plurality of second detection target sequences that are complementary to at least one detection probe nucleic acid sequence and non-complementary to said target nucleic acid sequence, under conditions where said target probe portion of said third nucleic acid molecule hybridizes directly to said cellular target nucleic acid sequence;
contacting said third nucleic acid molecule with a plurality of fourth nucleic acid molecules each comprising a detection probe portion complementary to said detection target sequences of said third nucleic acid molecule and a detectable moiety portion comprising at least one second detectable moiety either 5' or 3' to said detection probe portion, wherein said at least one second detectable moiety is incorporated into the nucleic acid molecule, under conditions such that said detection probe portion of said fourth nucleic acid molecule hybridizes to said second detection target sequences of said third nucleic acid molecule; and
detecting said at least one second detectable moiety.

5. The method of Claim 4, wherein said first and second cellular target nucleic acid sequences are part of the same molecule.

6. The method of Claim 5, wherein said first and second detectable moieties are the same.

## Patentansprüche

1. Verfahren zum Nachweisen einer ersten zellulären Zielnukleinsäuresequenz in situ in einer Gewebeprobe, umfassend:
Inkontaktbringen der Gewebeprobe mit einem ersten Nukleinsäuremolekül, das einen Zielsondenanteil, der eine zur ersten zellulären Zielnukleinsäuresequenz komplementäre Nukleinsäuresequenz umfasst, und einen Nachweiszielanteil, der mehrere erste Nachweiszielsequenzen umfasst, die zu wenigstens einer Nachweissondennukleinsäuresequenz komplementär und zur Zielnukleinsäuresequenz nicht komplementär sind, umfasst, unter Bedingungen, bei denen der Zielsondenanteil des ersten Nukleinsäuremoleküls direkt an die zelluläre Zielnukleinsäuresequenz hybridisiert;
Inkontaktbringen des ersten Nukleinsäuremoleküls mit mehreren zweiten Nukleinsäuremolekülen, die jeweils einen Nachweissondenanteil, der zu den Nachweiszielsequenzen des ersten Nukleinsäuremoleküls komplementär ist, und einen Nachweisbare-Gruppierung-Anteil, der wenigstens eine erste nachweisbare Gruppierung entweder 5' oder 3' zum Nachweissondenanteil umfasst, wobei die wenigstens eine erste nachweisbare Gruppierung in das Nukleinsäuremolekül eingebaut ist, umfassen, unter solchen Bedingungen, dass der Nachweissondenanteil des zweiten Nukleinsäuremoleküls an die ersten Nachweiszielsequenzen des ersten Nukleinsäuremoleküls hybridisiert; und
Nachweisen der wenigstens einen ersten nachweisbaren Gruppierung.

2. Verfahren nach Anspruch 1, wobei der Zielsondenanteil des ersten Nukleinsäuremoleküls und die Zielnukleinsäuresequenz und der Nachweissondenanteil der zweiten Nukleinsäure und die Nachweiszielsequenzen des ersten Nukleinsäuremoleküls Schmelzpunkte aufweisen, die innerhalb von etwa 10 Graden Celsius liegen.

3. Verfahren nach Anspruch 1, wobei die zelluläre Zielnukleinsäuresequenz aus der aus genomischer DNA, nukleärer DNA, RNA, mRNA und cytoplasmatischer Nukleinsäure bestehenden Gruppe ausgewählt ist.

4. Verfahren nach Anspruch 1, ferner umfassend Inkontaktbringen der Gewebeprobe mit einem dritten Nukleinsäuremolekül, das einen Zielsondenanteil, der eine zu einer zweiten zellulären Zielnukleinsäuresequenz komplementäre Nukleinsäuresequenz umfasst, und einen Nachweiszielanteil, der mehrere zweite Nachweiszielsequenzen umfasst, die zu wenigstens einer Nachweissondennukleinsäuresequenz komplementär und zur Zielnukleinsäuresequenz nicht komplementär sind, umfasst, unter Bedingungen, bei denen der Zielsondenanteil des dritten Nukleinsäuremoleküls direkt an die zelluläre Zielnukleinsäuresequenz hybridisiert;
Inkontaktbringen des dritten Nukleinsäuremoleküls mit mehreren vierten Nukleinsäuremolekülen, die jeweils einen Nachweissondenanteil, der zu den Nachweiszielsequenzen des dritten Nukleinsäuremoleküls komplementär ist, und einen Nachweisbare-Gruppierung-Anteil, der wenigstens eine zweite nachweisbare Gruppierung entweder 5' oder 3' zum Nachweissondenanteil umfasst, wobei die wenigstens eine zweite nachweisbare Gruppierung in das Nukleinsäuremolekül eingebaut ist, umfassen, unter solchen Bedingungen, dass der Nachweissondenanteil des vierten Nukleinsäuremoleküls an die zweiten Nachweiszielsequenzen des dritten Nukleinsäuremoleküls hybridisiert; und
Nachweisen der wenigstens einen zweiten nachweisbaren Gruppierung.

5. Verfahren nach Anspruch 4, wobei die erste und die zweite zelluläre Zielnukleinsäuresequenz Teil des gleichen Moleküls sind.

6. Verfahren nach Anspruch 5, wobei die erste und die zweite nachweisbare Gruppierung identisch sind.

## Revendications

1. Méthode pour détecter une première séquence d'acides nucléiques cible cellulaire in situ dans un échantillon de tissu comprenant :
la mise en contact dudit échantillon de tissu avec une première molécule d'acide nucléique comprenant une portion sonde cible comprenant une séquence d'acides nucléiques complémentaire de ladite première séquence d'acides nucléiques cible cellulaire et une portion cible de détection comprenant une pluralité de premières séquences cibles de détection qui sont complémentaires d'au moins une séquence d'acides nucléiques de sonde de détection et non complémentaires de ladite séquence d'acides nucléiques cible, dans des conditions où ladite portion sonde cible de ladite première molécule d'acide nucléique s'hybride directement à ladite séquence d'acides nucléiques cible cellulaire ;
la mise en contact de ladite première molécule d'acide nucléique avec une pluralité de deuxièmes molécules d'acide nucléique comprenant chacune une portion sonde de détection complémentaire desdites séquences cibles de détection de ladite première molécule d'acide nucléique et une portion groupement détectable comprenant au moins un premier groupement détectable soit en 5', soit en 3' sur ladite portion sonde de détection, dans laquelle ledit au moins un premier groupement détectable est incorporé dans la molécule d'acide nucléique, dans des conditions telles que ladite portion sonde de détection de ladite deuxième molécule d'acide nucléique s'hybride auxdites premières séquences cibles de détection de ladite première molécule d'acide nucléique ; et
la détection dudit au moins un premier groupement détectable.

2. Méthode de la revendication 1, dans laquelle ladite portion sonde cible de ladite première molécule d'acide nucléique et ladite séquence d'acides nucléiques cible et ladite portion sonde de détection dudit deuxième acide nucléique et lesdites séquences cibles de détection de ladite première molécule d'acide nucléique ont des points de fusion de l'ordre d'environ 10 degrés Celsius.

3. Méthode de la revendication 1, dans laquelle ladite séquence d'acides nucléiques cible cellulaire est sélectionnée dans le groupe constitué d'un ADN génomique, d'un ADN nucléaire, d'un ARN, d'un ARNm, et d'un acide nucléique cytoplasmique.

4. Méthode de la revendication 1, comprenant en outre la mise en contact de l'échantillon de tissu avec une troisième molécule d'acide nucléique comprenant une portion sonde cible comprenant une séquence d'acides nucléiques complémentaire d'une deuxième séquence d'acides nucléiques cible cellulaire et une portion cible de détection comprenant une pluralité de deuxièmes séquences cibles de détection qui sont complémentaires d'au moins une séquence d'acides nucléiques de sonde de détection et non complémentaires de ladite séquence d'acides nucléiques cible, dans des conditions où ladite portion sonde cible de ladite troisième molécule d'acide nucléique s'hybride directement à ladite séquence d'acides nucléiques cible cellulaire ;
la mise en contact de ladite troisième molécule d'acide nucléique avec une pluralité de quatrièmes molécules d'acide nucléique comprenant chacune une portion sonde de détection complémentaire desdites séquences cibles de détection de ladite troisième molécule d'acide nucléique et une portion groupement détectable comprenant au moins un deuxième groupement détectable soit en 5', soit en 3' sur ladite portion sonde de détection, dans laquelle ledit au moins un deuxième groupement détectable est incorporé dans la molécule d'acide nucléique, dans des conditions telles que ladite portion sonde de détection de ladite quatrième molécule d'acide nucléique s'hybride auxdites deuxièmes séquences cibles de détection de ladite troisième molécule d'acide nucléique ; et
la détection dudit au moins un deuxième groupement détectable.

5. Méthode de la revendication 4, dans laquelle lesdites premières et deuxièmes séquences d'acides nucléiques cibles cellulaires font partie de la même molécule.

6. Méthode de la revendication 5, dans laquelle lesdits premier et deuxième groupements détectables sont identiques.


### SEQUENCE LISTING

<110> Ventana Medical Systems, Inc.
   Farrell, Michael
   Jiang, Zeyu
   Day, William A. Jr.
<120> Polytag Probes
<130> VENTA-31371/WO-1/ORD
<150> US 61/308,670
   <151> 2010-02-26
<160> 85
<170> PatentIn version 3.5
<210> 1
   <211> 40
   <212> RNA
   <213> Artificial Sequence
<220>
   <223> Synthetic
<400> 1
   caucagcagg acgcacugac caccaugaag gugcucuucu 40
<210> 2
   <211> 48
   <212> RNA
   <213> Artificial Sequence
<220>
   <223> Synthetic
<400> 2
   agaacaagaa uacuaccguc augcacuuga uccggcacgg ucacuagu 48
<210> 3
   <211> 47
   <212> RNA
   <213> Artificial Sequence
<220>
   <223> Synthetic
<400> 3
   uuacaccuca ccgacaauag aagaucgucc uggcacugaa cuugccu 47
<210> 4
   <211> 39
   <212> RNA
   <213> Artificial Sequence
<220>
   <223> Synthetic
<400> 4
   uccgcaguaa cgcuuaaucg cuccagacga cacccaugg 39
<210> 5
   <211> 65
   <212> DNA
   <213> Artificial Sequence
<220>
   <223> Synthetic
<400> 5
   ugcgcaagaa ctcatggcta acggacaccg caauacaaug auaccugucg ccuucgcgua 60
   ugcau 65
<210> 6
   <211> 104
   <212> RNA
   <213> Artificial Sequence
<220>
   <223> Synthetic
<400> 6
   cacagaacua aacagaagca uucucagaac ccucuucgug auguuugcau ucaacucaca 60
   gugccaucag caggacgcac ugaccaccau gaaggugcuc uucu 104
<210> 7
   <211> 120
   <212> RNA
   <213> Artificial Sequence
<220>
   <223> Synthetic
<400> 7
   ugggcauggg ucagaaggau uccuaugugg gcgacgaggc ccagagcaag agaggcaucc 60
   ucacccugaa guaccccauc caucagcagg acgcacugac caccaugaag gugcucuucu 120
<210> 8
   <211> 120
   <212> RNA
   <213> Artificial Sequence
<220>
   <223> Synthetic
<400> 8
   gagcacggca ucgucaccaa cugggacgac auggagaaaa ucuggcacca caccuucuac 60
   aaugagcugc guguggcucc caucagcagg acgcacugac caccaugaag gugcucuucu 120
<210> 9
   <211> 122
   <212> DNA
   <213> Artificial Sequence
<220>
   <223> Synthetic
<400> 9
   cgaggagcac cccgugcugc tugaccgagg ccccccuuga accccaaggc caaccgcgag      60
   aagaugaccc agaucatguu ugcaucagca ggacgcacug accaccauga aggugcucuu     120
   cu                                                                   122
<210> 10
   <211> 120
   <212> RNA
   <213> Artificial Sequence
<220>
   <223> Synthetic
<400> 10
   agaccuucaa caccccagcc auguacguug cuauccaggc ugugcuaucc cuguacgccu      60
   cuggccguac cacuggcauc caucagcagg acgcacugac caccaugaag gugcucuucu     120
<210> 11
   <211> 120
   <212> RNA
   <213> Artificial Sequence
<220>
   <223> Synthetic
<400> 11
   gugauggacu ccggugacgg ggucacccac acugugccca ucuacgaggg guaugcccuc      60
   ccccaugcca uccugcgucu caucagcagg acgcacugac caccaugaag gugcucuucu     120
<210> 12
   <211> 120
   <212> RNA
   <213> Artificial Sequence
<220>
   <223> Synthetic
<400> 12
   ggaccuggcu ggccgggacc ugacugacua ccucaugaag auccucaccg agcgcggcua      60
   cagcuucacc accacggccg caucagcagg acgcacugac caccaugaag gugcucuucu     120
<210> 13
   <211> 121
   <212> DNA
   <213> Artificial Sequence
<220>
   <223> Synthetic
<400> 13
   agcgggaaau cgugcgugac auuaaggaga agcugugcua cgucgccccu ggacuucgag 60
   caagagaugg ccacggcugc ucaucagcag gacgcacuga ccaccaugaa ggugcucuuc 120
   u 121
<210> 14
   <211> 120
   <212> DNA
   <213> Artificial Sequence
<220>
   <223> Synthetic
<400> 14
   uccagcuccu cccuggagaa gagcuacgag cugcctgacg gccaggucau caccauuggc 60
   aaugagcggu uccgcugccc caucagcagg acgcacugac caccaugaag gugcucuucu 120
<210> 15
   <211> 120
   <212> RNA
   <213> Artificial Sequence
<220>
   <223> Synthetic
<400> 15
   ugaggcacuc uuccagccuu ccuuccuggg cauggagucc uguggcaucc acgaaacuac 60
   cuucaacucc aucaugaagu caucagcagg acgcacugac caccaugaag gugcucuucu 120
<210> 16
   <211> 121
   <212> DNA
   <213> Artificial Sequence
<220>
   <223> Synthetic
<400> 16
   gugacgugga cauccgcaaa gaccuguacg ccaacacagu gcugtucugg cggcaccacc 60
   auguacccug gcauugccga ccaucagcag gacgcacuga ccaccaugaa ggugcucuuc 120
   u 121
<210> 17
   <211> 120
   <212> RNA
   <213> Artificial Sequence
<220>
   <223> Synthetic
<400> 17
   aggaugcaga aggagaucac ugcccuggca cccagcacaa ugaagaucaa gaucauugcu 60
   ccuccugagc gcaaguacuc caucagcagg acgcacugac caccaugaag gugcucuucu 120
<210> 18
   <211> 122
   <212> RNA
   <213> Artificial Sequence
<220>
   <223> Synthetic
<400> 18
   cguguggauc ggcggcucca uccuggccuc gcuuguccac cuuccagcag auguggauca 60
   gcaagcagga guuaugacga gucaucagca ggacgcacug accaccauga aggugcucuu 120
   cu 122
<210> 19
   <211> 128
   <212> RNA
   <213> Artificial Sequence
<220>
   <223> Synthetic
<400> 19
   cggaacugag gccaugauua agagggacgg ccgggggcau ucguauugcg ccgcuagagg 60
   ugaaauucuu ggaccggcgc agaacaagaa uacuaccguc augcacuuga uccggcacgg 120
   ucacuagu 128
<210> 20
   <211> 127
   <212> RNA
   <213> Artificial Sequence
<220>
   <223> Synthetic
<400> 20
   cggaacugag gccaugauua agagggacgg ccgggggcau ucguauugcg ccgcuagagg 60
   ugaaauucuu ggaccggcgc uuacaccuca ccgacaauag aagaucgucc uggcacugaa 120
   cuugccu 127
<210> 21
   <211> 145
   <212> DNA
   <213> Artificial Sequence
<220>
   <223> Synthetic
<400> 21 auaccugucg ccuucgcgua ugcau 145
<210> 22
   <211> 70
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<400> 22
   ntattttnta ttttntattt tntattttnt agaagagcac cttcatggtg gtcagtgcgt      60
   cctgctgatg                                                              70
<210> 23
   <211> 105
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<400> 23
   ntattttnta ttttntattt tntattttnt attttntatt ttntattttn tattttntat      60
   tttntagaag agcaccttca tggtggtcag tgcgtcctgc tgatg                     105
<210> 24
   <211> 140
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<220>
   <221> misc_feature
   <222> (71)..(71)
   <223> n is an abasic moiety containing a hapten
<220>
   <221> misc_feature
   <222> (78)..(78)
   <223> n is an abasic moiety containing a hapten
<220>
   <221> misc_feature
   <222> (85)..(85)
   <223> n is an abasic moiety containing a hapten
<220>
   <221> misc_feature
   <222> (92)..(92)
   <223> n is an abasic moiety containing a hapten
<220>
   <221> misc_feature
   <222> (99)..(99)
   <223> n is an abasic moiety containing a hapten
<400> 24
   ntattttnta ttttntattt tntattttnt attttntatt ttntattttn tattttntat 60
   tttntatttt ntattttnta ttttntattt tntattttnt agaagagcac cttcatggtg 120
   gtcagtgcgt cctgctgatg 140
<210> 25
   <211> 77
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<400> 25
   ntattttnta ttttntattt tntattttna ctagtgaccg tgccggatca agtgcatgac 60
   ggtagtattc ttgttct 77
<210> 26
   <211> 112
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<400> 26
   ntattttnta ttttntattt tntattttnt attttntatt ttntattttn tattttntat 60
   tttnactagt gaccgtgccg gatcaagtgc atgacggtag tattcttgtt ct 112
<210> 27
   <211> 147
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<220>
   <221> misc_feature
   <222> (71)..(71)
   <223> n is an abasic moiety containing a hapten
<220>
   <221> misc_feature
   <222> (78)..(78)
   <223> n is an abasic moiety containing a hapten
<220>
   <221> misc_feature
   <222> (85)..(85)
   <223> n is an abasic moiety containing a hapten
<220>
   <221> misc_feature
   <222> (92)..(92)
   <223> n is an abasic moiety containing a hapten
<220>
   <221> misc_feature
   <222> (99)..(99)
   <223> n is an abasic moiety containing a hapten
<400> 27
   ntattttnta ttttntattt tntattttnt attttntatt ttntattttn tattttntat 60
   tttntatttt ntattttnta ttttntattt tntattttna ctagtgaccg tgccggatca 120
   agtgcatgac ggtagtattc ttgttct 147
<210> 28
   <211> 76
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<400> 28
   ntattttnta ttttntattt tntattttna ggcaagttca gtgccaggac gatcttctat 60
   tgtcggtgag gtgtaa 76
<210> 29
   <211> 111
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<400> 29
   ntattttnta ttttntattt tntattttnt attttntatt ttntattttn tattttntat 60
   tttnaggcaa gttcagtgcc aggacgatct tctattgtcg gtgaggtgta a 111
<210> 30
   <211> 146
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<220>
   <221> misc_feature
   <222> (71)..(71)
   <223> n is an abasic moiety containing a hapten
<220>
   <221> misc_feature
   <222> (78)..(78)
   <223> n is an abasic moiety containing a hapten
<220>
   <221> misc_feature
   <222> (85)..(85)
   <223> n is an abasic moiety containing a hapten
<220>
   <221> misc_feature
   <222> (92)..(92)
   <223> n is an abasic moiety containing a hapten
<220>
   <221> misc_feature
   <222> (99)..(99)
   <223> n is an abasic moiety containing a hapten
<400> 30
   ntattttnta ttttntattt tntattttnt attttntatt ttntattttn tattttntat 60
   tttntatttt ntattttnta ttttntattt tntattttna ggcaagttca gtgccaggac 120
   gatcttctat tgtcggtgag gtgtaa 146
<210> 31
   <211> 68
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<400> 31
   ntattttnta ttttntattt tntattttnc catgggtgtc gtctggagcg attaagcgtt 60
   actgcgga 68
<210> 32
   <211> 103
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<400> 32
   ntattttnta ttttntattt tntattttnt attttntatt ttntattttn tattttntat 60
   tttnccatgg gtgtcgtctg gagcgattaa gcgttactgc gga 103
<210> 33
   <211> 138
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<220>
   <221> misc_feature
   <222> (71)..(71)
   <223> n is an abasic moiety containing a hapten
<220>
   <221> misc_feature
   <222> (78)..(78)
   <223> n is an abasic moiety containing a hapten
<220>
   <221> misc_feature
   <222> (85)..(85)
   <223> n is an abasic moiety containing a hapten
<220>
   <221> misc_feature
   <222> (92)..(92)
   <223> n is an abasic moiety containing a hapten
<220>
   <221> misc_feature
   <222> (99)..(99)
   <223> n is an abasic moiety containing a hapten
<400> 33
   ntattttnta ttttntattt tntattttnt attttntatt ttntattttn tattttntat 60
   tttntatttt ntattttnta ttttntattt tntattttnc catgggtgtc gtctggagcg 120
   attaagcgtt actgcgga 138
<210> 34
   <211> 77
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<400> 34
   ntattttnta ttttntattt tntattttnc gaaggcgaca ggtatcattg tattgcggtg 60
   tccgttagcc atgagtt 77
<210> 35
   <211> 112
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<400> 35
   ntattttnta ttttntattt tntattttnt attttntatt ttntattttn tattttntat 60
   tttncgaagg cgacaggtat cattgtattg cggtgtccgt tagccatgag tt 112
<210> 36
   <211> 147
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<220>
   <221> misc_feature
   <222> (71)..(71)
   <223> n is an abasic moiety containing a hapten
<220>
   <221> misc_feature
   <222> (78)..(78)
   <223> n is an abasic moiety containing a hapten
<220>
   <221> misc_feature
   <222> (85)..(85)
   <223> n is an abasic moiety containing a hapten
<220>
   <221> misc_feature
   <222> (92)..(92)
   <223> n is an abasic moiety containing a hapten
<220>
   <221> misc_feature
   <222> (99)..(99)
   <223> n is an abasic moiety containing a hapten
<400> 36
   ntattttnta ttttntattt tntattttnt attttntatt ttntattttn tattttntat 60
   tttntatttt ntattttnta ttttntattt tntattttnc gaaggcgaca ggtatcattg 120
   tattgcggtg tccgttagcc atgagtt 147
<210> 37
   <211> 25
   <212> DNA
   <213> Artificial Sequence
<220>
   <223> Synthetic
<400> 37
   agaagtatcg ttccgatcta acgcg 25
<210> 38
   <211> 25
   <212> DNA
   <213> Artificial Sequence
<220>
   <223> Synthetic
<400> 38
   acgctattac gattacgacg tgcga 25
<210> 39
   <211> 25
   <212> DNA
   <213> Artificial Sequence
<220>
   <223> Synthetic
<400> 39
   tacgatcgca tcgagtcgca gatat 25
<210> 40
   <211> 25
   <212> DNA
   <213> Artificial Sequence
<220>
   <223> Synthetic
<400> 40
   cgcacgcata gttagtcgga tatac 25
<210> 41
   <211> 25
   <212> DNA
   <213> Artificial Sequence
<220>
   <223> Synthetic
<400> 41
   ctagctccga tccgtgataa cgtgc 25
<210> 42
   <211> 25
   <212> DNA
   <213> Artificial Sequence
<220>
   <223> Synthetic
<400> 42
   atgttacgac cggcgatctt atacg 25
<210> 43
   <211> 30
   <212> DNA
   <213> Artificial Sequence
<220>
   <223> Synthetic
<400> 43
   gtacatcctc cggttgcgaa tatagcgaac 30
<210> 44
   <211> 20
   <212> DNA
   <213> Artificial Sequence
<220>
   <223> Synthetic
<400> 44
   ctagatctct cgagacatgc 20
<210> 45
   <211> 25
   <212> DNA
   <213> Artificial Sequence
<220>
   <223> Synthetic
<400> 45
   ttctagatct ctcgagacat gcaca 25
<210> 46
   <211> 150
   <212> DNA
   <213> Artificial Sequence
<220>
   <223> Synthetic
<400> 46
   tccgtgataa cgtgcgatat ctagctccga tccgtgataa cgtgcgatat ctagctcacg       60
   tccgtgataa cgtgcgatat ctagctccac tccgtgataa cgtgcgatat ctagctcgac      120
   tccgtgataa cgtgcgatat ctagctcctg                                       150
<210> 47
   <211> 127
   <212> DNA
   <213> Artificial Sequence
<220>
   <223> Synthetic
<220>
   <221> misc_feature
   <222> (1)..(1)
   <223> n is an abasic moiety containing a hapten
<220>
   <221> misc_feature
   <222> (8)..(8)
   <223> n is an abasic moiety containing a hapten
<220>
   <221> misc_feature
   <222> (15)..(15)
   <223> n is an abasic moiety containing a hapten
<220>
   <221> misc_feature
   <222> (22)..(22)
   <223> n is an abasic moiety containing a hapten
<220>
   <221> misc_feature
   <222> (29)..(29)
   <223> n is an abasic moiety containing a hapten
<220>
   <221> misc_feature
   <222> (36)..(36)
   <223> n is an abasic moiety containing a hapten
<220>
   <221> misc_feature
   <222> (43)..(43)
   <223> n is an abasic moiety containing a hapten
<220>
   <221> misc_feature
   <222> (50)..(50)
   <223> n is an abasic moiety containing a hapten
<220>
   <221> misc_feature
   <222> (57)..(57)
   <223> n is an abasic moiety containing a hapten
<220>
   <221> misc_feature
   <222> (64)..(64)
   <223> n is an abasic moiety containing a hapten
<220>
   <221> misc_feature
   <222> (71)..(71)
   <223> n is an abasic moiety containing a hapten
<220>
   <221> misc_feature
   <222> (78)..(78)
   <223> n is an abasic moiety containing a hapten
<220>
   <221> misc_feature
   <222> (85)..(85)
   <223> n is an abasic moiety containing a hapten
<220>
   <221> misc_feature
   <222> (92)..(92)
   <223> n is an abasic moiety containing a hapten
<220>
   <221> misc_feature
   <222> (99)..(99)
   <223> n is an abasic moiety containing a hapten
<400> 47
   ntattttnta ttttntattt tntattttnt attttntatt ttntattttn tattttntat 60
   tttntatttt ntattttnta ttttntattt tntattttnt gagctagata tcgcacgtta 120
   tcacgga 127
<210> 48
   <211> 24
   <212> DNA
   <213> Artificial Sequence
<220>
   <223> Synthetic
<400> 48
   tgcctgcagg tcgacaggag ctag 24
<210> 49
   <211> 80
   <212> DNA
   <213> Artificial Sequence
<220>
   <223> Synthetic
<400> 49
   accgtctcga ttaccgagag tgcgctgaac cggaatgtac gatcaattag gcgtcgtccg 60
atcgtagatt actaactgct 80
<210> 50
   <211> 206
   <212> DNA
   <213> Artificial Sequence
<220>
   <223> Synthetic
<400> 50
   ggctgctcct ctttaccttt ctgtcactct cttagaacgt gggagtagac ggatgcgaaa 60
   atgtccgtag tttgggtgac tataacattt aaccctggtc aggttgctag gtcatatatt 120
   ttgtgtttcc tttctgtgta ttcaacctag ggtgtgtttg gctagacgga actcttgcct 180
   ggttgcaagt gtcaagccac cgattg 206
<210> 51
   <211> 217
   <212> DNA
   <213> Artificial Sequence
<220>
   <223> Synthetic
<400> 51
   attgctgctc accgttttta ggtttcaggt cctctgacac cttttggtat cgttaatttt 60
   actgatttgt gtagaatgtc agttgtattt taccagctaa tatctagaaa tgctggcaag 120
   aggggtttac tccagcttta gattgtaggt atgttagctt ttttcataca gtgtattaaa 180
   tttactgagt cagcttgctg aataagacag aagccca 217
<210> 52
   <211> 202
   <212> DNA
   <213> Artificial Sequence
<220>
   <223> Synthetic
<400> 52
   gggcttcaaa agttagtggt catcgaaaag cattaatctt tgcagtttca ggtacaacac 60
   attggttttg attagggatg gggatggggc cctctttttg cagaatgggg aaagtattga 120
   caggaattga gagctattgg taggccagtg tataaggtat gtgaaaacag aattaagtta 180
   ttggtctgaa gtgactgaag ca 202
<210> 53
   <211> 202
   <212> DNA
   <213> Artificial Sequence
<220>
   <223> Synthetic
<400> 53
   agcgtatgtt ggtctctaca catgaaattt gtgtgactta aaactttctc taaaactgta 60
   cttttagtta tgatatgcat agaaagcagt atcaaatatt gcgtcaaatg actaataaca 120
   cttaatttct agagttgtgg ttttattgag ccaaaagttg atatgaaaaa aagtcagtaa 180
   ggaaagtcag tgaagtgctt gc 202
<210> 54
   <211> 217
   <212> DNA
   <213> Artificial Sequence
<220>
   <223> Synthetic
<400> 54
   ttgctgccag tgtaaaagtt tgcacagcag tatagtcatc aatgcagatt tacattgctt 60
   ataatatact aagtaaatac taaatgatta aagataataa aatatggtga ggtataacca 120
   ccttcatttt aaacttagtt ttagaagata gtaaagaaag attcctttat taccttttta 180
   gaattttatt tttaataaca tgggaaaggc aactggt 217
<210> 55
   <211> 204
   <212> DNA
   <213> Artificial Sequence
<220>
   <223> Synthetic
<400> 55
   aatttgtgag actggggtca gtcagttctg ttttacaatt gctttctatt tggtagcttt        60
   gaaattaatt tagttgctta tcagagagaa taatgttgag gttagactaa ccttaaattg       120
   gtaaggcttt gctgagcaaa ctgataactg taagtctttt atagggtgca ttactgccac       180
   atatacgttc ttccataggt ggtt                                              204
<210> 56
   <211> 215
   <212> DNA
   <213> Artificial Sequence
<220>
   <223> Synthetic
<400> 56
   tggtccatgt ctaggttgta gaattgaatt gtgcattttg gcatctgagc acagctgagt        60
   tttctaaatc aatctctctc cttgcaccta gtttttgctt tagatcacta cctaagactt       120
   actgttgatt taatattaga gcacttaagc atagctttga cttttatttc ctttgatttt       180
   tgtagatttt caggctgaag tacaataagg ttctc                                  215
<210> 57
   <211> 200
   <212> DNA
   <213> Artificial Sequence
<220>
   <223> Synthetic
<400> 57
   aaattccctc tctttgtgag acttcttttt gagtattctg gttactctaa actgattgga        60
   gatgaaatta gatagaattg aaaactgtac ttttaaaatg aaattttggg gatgtcatta       120
   agcttgattt tttaggtttt ttttttagtg tgtattataa attattttac actgattgtc       180
   agcgataaaa tggaatgcct                                                   200
<210> 58
   <211> 218
   <212> DNA
   <213> Artificial Sequence
<220>
   <223> Synthetic
<400> 58
   aaaatcagta cctttgcccc caggtgtgat atttaagaag gtcaacttac taaatcagtg        60
   atggagttag tcctaacatc tgggtgttct gactgctgct aggccagtat tctttatatg       120
   ataataagaa ctttgtccac agaagatatc cctaataaca aaaaaggttt atttgaagag       180
   gactcatgtg ttctttggct gattgtgaaa gtgttgct                               218
<210> 59
   <211> 210
   <212> DNA
   <213> Artificial Sequence
<220>
   <223> Synthetic
<400> 59
   agttgttgaa ctgttgggag ttacttttct cttactattt tgttatttaa tgtattcttt        60
   gaccttatgc ttttttattc taaagctgct tttattatag tcagatatga tgaagttaaa       120
   tgtacaatgt aaaattgcaa atttccaacg agctatacaa acttaaatat ttctaagtaa       180
   agaaaatagg gctgactcta aggttctttg                                        210
<210> 60
   <211> 202
   <212> DNA
   <213> Artificial Sequence
<220>
   <223> Synthetic
<400> 60
   taggttagcc cagagatggg aagatgccaa gaaggtagct ttagtggatt ctgaattttt        60
   tggttttgtt ttgtttttag ggcaggcaaa tgtaattaca aaagggttct aggaatagat       120
   tgctgtgatt ttttttctgt ttgcatgatt ttacagtttg ctttgcctct cacttttgaa       180
   tgcagaataa aatgtcaagg cc                                                202
<210> 61
   <211> 206
   <212> DNA
   <213> Artificial Sequence
<220>
   <223> Synthetic
<400> 61
   tgcactttgt cgttgcctta attaaatggt gaaatcatca gaaatattta ttttcctata 60
   cttatacatt tattaagctt gtttccattt ttttattttg tgatttttta agtggattta 120
   agataaccta aacattagag aggattttca tggttttgat tcatgaaatc ataatgttat 180
   acaaacctaa ctgaagtgtt agagcc 206
<210> 62
   <211> 205
   <212> DNA
   <213> Artificial Sequence
<220>
   <223> Synthetic
<400> 62
   tggctgagaa ctaaagattg tgtaataaac gcctggcctt cagtcatttg gttttttttt 60
   tccctcgatt gtttggatag ttaactggac atcatgtttt aacttgagaa attaagttat 120
   acaagatttt gatattttaa actagttttc ctaactggtt gagatatata agaatttagt 180
   attacaggac tcaatcaggg aactg 205
<210> 63
   <211> 208
   <212> DNA
   <213> Artificial Sequence
<220>
   <223> Synthetic
<400> 63
   ggtgagaact gaattggagg ctatgaaaaa aatacctttt gggcctttct gaatagacat 60
   atatacataa attatatctc ttacattaag tgaggcacat atgtaggtga gatttttacc 120
   tgaatattaa aagtttaaaa gtcgttacct attctgttta cttaatagta tttaaagggt 180
   gtgagaggtg ttatgtgttt ctgtccct 208
<210> 64
   <211> 216
   <212> DNA
   <213> Artificial Sequence
<220>
   <223> Synthetic
<400> 64
   ggttaatcac ctctggcaaa ataaatgata aaagcatagc ttttgtaagc agaatgatat 60
   tacagaagtt aacttataaa tctaagtgta ttaaagacac ttaggaaatt tatgataatg 120
   ctgggtcagc attacagttt taacttttta cagtttttca tatgcttttt ttgtgatttt 180
   gctgtagaaa attaacagtt ggcatttggc ttagtt 216
<210> 65
   <211> 206
   <212> DNA
   <213> Artificial Sequence
<220>
   <223> Synthetic
<400> 65
   tgttgccaaa tgaacgagtt tgtagtattg ctaacaagga gaagaattac tagcaagtct 60
   tgatgttact tttgaagagt gtgatgattg catttaggaa gatatctaaa cttctgtttc 120
   aaagcaaaaa gtatgtgcaa atttcttact catgacaaat tcatataata taaaaacatg 180
   aaagttgtga ggtcaggttg tttgga 206
<210> 66
   <211> 212
   <212> DNA
   <213> Artificial Sequence
<220>
   <223> Synthetic
<400> 66
   tgctcacaag aaccctaact gtgtgttact tgaaagcact gatggaaatc agggaaaaag 60
   ctccagaagt tcctacgaaa taaaattaaa tgataaagtc ctggtatctg ctaacttgcc 120
   ttccattcct gttatctttt cttcttagtc tgacttcatt aattctttca ccctggctac 180
   tggtttagct cagtgtttta tgagccaggc ag 212
<210> 67
   <211> 208
   <212> DNA
   <213> Artificial Sequence
<220>
   <223> Synthetic
<400> 67
   aggaaggtga gaatctgaag aaaatgaaac cttaaaaaga ttgaattcct ggactccatt 60
   taaaggagta aatagctcac gaacaagact tgctgctctg caaagtcttc catgttgatc 120
   ctggtctttg actccttatc tgtctgatta aattgaattc gctgccgtgg catccttaaa 180
   gctggacctt actttgtcag tcctgcct 208
<210> 68
   <211> 200
   <212> DNA
   <213> Artificial Sequence
<220>
   <223> Synthetic
<400> 68
   agtgcagtaa aagtgcagtg tccaaatagc ccttgtaaca aaacctttct ctttctcctg 60
   ggtgccaatt tgacatttaa tcagttttgt ttctagcagt gttcaattta ttagattata 120
   agtctttttt ttctttatat tattctaaga tcaaaaatat ataaagatat acacaggagt 180
   cctgctgcta cctgttcttg 200
<210> 69
   <211> 214
   <212> DNA
   <213> Artificial Sequence
<220>
   <223> Synthetic
<400> 69
   cttgtctttt caggcaggtg tcaattttgg ggttttgttt tgatttttgg tttttgacat 60
   aaagtacttt agttctgtga tgtataaacc gtgagtttct gtttttctca tatacctgaa 120
   tactgtccat gtggaagtta ccttttatct ttaccagtat taacacataa atggttatac 180
   ataaatacat tgaccacctt ttattactcc agct 214
<210> 70
   <211> 220
   <212> DNA
   <213> Artificial Sequence
<220>
   <223> Synthetic
<400> 70
   tgatgggaac agcaggttga tatagcttgt gataacactt ctaaagaaaa agcaatgagc 60
   catagaaaaa agaaaaagat acattttgaa ttaaggaaga tggtgaatct gggaagtgag 120
   cagtacagtc accagacgtg tatcctctcc tatggtacag aagtgtttat tgggtctctt 180
   tatggcctgc atgatatatc ccacaagatg acctacttca 220
<210> 71
   <211> 215
   <212> DNA
   <213> Artificial Sequence
<220>
   <223> Synthetic
<400> 71
   acctttatgc ctctgaagga aaagatttat acattcagct tgtaattagt aatcaagact 60
   gaggtttagt ctatctagct tcacaatcta tctagtttgt tttgtctagc catatgattt 120
   cttcaaatat gccatttctt aaaaaaaaat gttttatgta tcccgattaa tatttagcca 180
   gtggttcttt tagccgatgg atcttgtcac ctctt 215
<210> 72
   <211> 210
   <212> DNA
   <213> Artificial Sequence
<220>
   <223> Synthetic
<400> 72
   ttttgattgg gggataattg gccaataaag ctttgatagc ctctattgcc caggcccctc 60
   ctcttctttt atgagagaaa ggatgaacag tgaccagaaa taaaggtatt gtttttttct 120
   atcaactaaa atggaaataa ataattccta agtaatttgc ctgttaggat taaagtctcc 180
   aagagaatgg ctgtgcctag tacctaagtg 210
<210> 73
   <211> 200
   <212> DNA
   <213> Artificial Sequence
<220>
   <223> Synthetic
<400> 73
   acttctcctt ttgaggttac cgcctacgat tgggaattaa tgtaaaaaat aagccaaaag 60
   aaagtgaggg aaaagtgaac caagctgtaa tttttttact cttttttatt gttgttgtta 120
   ttgttgctgt tttttactat cttgattgca acagtttggc ttatatatat agcatttgga 180
   attgacagta agaaagccac 200
<210> 74
   <211> 205
   <212> DNA
   <213> Artificial Sequence
<220>
   <223> Synthetic
<400> 74
   tgcttttcct tccctaatcc ctcaggggtg ggatagagag cacagtggcc tcccagggag 60
   gtagaagctg ctccagacta acaatcagag ctgccagttc ttaatcccca agaccgccag 120
   acttcacaaa gacataccga ggtctgtgct gtcagtgccc cactactaca ctcccttaag 180
   tagccccaca ttcttgtgct tgttt 205
<210> 75
   <211> 216
   <212> DNA
   <213> Artificial Sequence
<220>
   <223> Synthetic
<400> 75
   gatattttgc agcatgtgaa gctttttaaa aagttaggct tattgaagta taatttacac 60
   acaaagtaca aaaaaaaaaa gactgtgttc tcaaatctgt gagtcattaa tgggtttaga 120
   tgtttatata ttgaaattat tggaagtaag gtatgtttat attagaaaga tttgtagtct 180
   agattatcca agttttggga gtattacctc tctgct 216
<210> 76
   <211> 220
   <212> DNA
   <213> Artificial Sequence
<220>
   <223> Synthetic
<400> 76
   ttttccgcct ttccattgtg tcagacttat aaggcaatca gccaactgtg ggcatgaaat 60
   ccttgggagg aaagagaagg aagtgggagg ggcagccatg gtgaatgttt ccctaagtta 120
   tagtcaagtt ctttgagaga acataacctc atcccctttt taaactgttg taatactttc 180
   ttttaaatag attgtttatt ctcctgcaag tctcacagtt 220
<210> 77
   <211> 200
   <212> DNA
   <213> Artificial Sequence
<220>
   <223> Synthetic
<400> 77
   aatctctgac tctcctgtac cttgtcctca ctaggattcg gtatccacgg caaaaagatc 60
   tattaatagt tggtatcagg cctgtacatg tgttaagaga aagatgagga aagaagtatc 120
   tgcttctaat ctcttgaaat tatctccaaa ttgaaatggt attttggttg cctaacagcc 180
   tgaagatgac aaatatcccc 200
<210> 78
   <211> 212
   <212> DNA
   <213> Artificial Sequence
<220>
   <223> Synthetic
<400> 78
   ggttctcttt gttaaagcag gcatttttca gatttgtctt ttgtcctagg gcatggtttt 60
   taacttcaag gttgcccttt ccaatgtctc agctaagtat ctggggtgtt ccatgaggtc 120
   tcttccactt tgcctaggcc agaactccag cttctcccag tattatattt cgttacctct 180
   ggcgtcatct ccgttatgct ttcagatcct gc 212
<210> 79
   <211> 207
   <212> DNA
   <213> Artificial Sequence
<220>
   <223> Synthetic
<400> 79
   tgttggcaca gattcatgtt acttgatctg ctttaaatga cttggcatct agcccatatt 60
   tgagcccata accgtgtggt aatttgaagt gtaattcaca gtagagcttc tgttaaagca 120
   ctaatagcat cttccatgga ggtatacttc agagtgaata taattttgtt tatcctgtgt 180
   ctctagagct attgactgaa aaagctg 207
<210> 80
   <211> 211
   <212> DNA
   <213> Artificial Sequence
<220>
   <223> Synthetic
<400> 80
   agagttttaa ggactgccca cctgattgat agagctagtt gaccttatct ttaacttttt 60
   gtttttcttt tgactttggg agtagagatg tgaaaaggta aaaaggaagg aaggaagaga 120
   aaacttaact ctttttgccc atgaagactg tttttccttc tcaaaatatt gactattttc 180
   tgatttgtaa aaatcggcac ataaaacgtg t 211
<210> 81
   <211> 210
   <212> DNA
   <213> Artificial Sequence
<220>
   <223> Synthetic
<400> 81
   aggggtcttt ctcttttcct gataaacctc tcctacaaag agccttgttg cggataccat 60
   agtgtttctt tggaggaaaa taaaaactac aaagctttgt attttttgca caactggatt 120
   cagaatataa gtaataaaaa aggacaagaa ctttcaaaag ctagaagcca ttaaactgag 180
   tcacttcagg gttagactat cagaactggg 210
<210> 82
   <211> 216
   <212> DNA
   <213> Artificial Sequence
<220>
   <223> Synthetic
<400> 82
   aggacaccaa agacaaattc ggcctttttc aaaattttat tctagtttaa catattcaaa 60
   gaaagggaag gaaattcttt tcattcctgt gtgtagtgac ttcctgcttt aagaacttag 120
   gacttcagct gtactatcag tattgtaggc cacttaacat tattatggtt aaagttggca 180
   ttggagagag cctaggaacc taactgcctg tttgtt 216
<210> 83
   <211> 209
   <212> DNA
   <213> Artificial Sequence
<220>
   <223> Synthetic
<400> 83
   gaccatccac tgtttatgcc aatattccct ttacgttttg cttttttgct tgttcgtttt 60
   aacctctcca aattttactg acttcagaag tttctagaac taagttatag catgttttga 120
   gttctaatgt cactttccga tcttctttac cttttttcta cctctgtttg tatttctggt 180
   tctggttaag tgagtctggt aagcagcag 209
<210> 84
   <211> 200
   <212> DNA
   <213> Artificial Sequence
<220>
   <223> Synthetic
<400> 84
   gagacttatc actaccaaac cacaaagaat ttaaaagaaa ctgtcagtag gtataggtgg 60
   aaggagggca tttatcagag attttaattt aagaagaaag tcttcatcct tatcctacca 120
   acccccattc cctgagcata tttatcatta ctagtcccag catatttgct cccatatttc 180
   ctatgcttac ctgtgaagat 200
<210> 85
   <211> 203
   <212> DNA
   <213> Artificial Sequence
<220>
   <223> Synthetic
<400> 85
   cattacttcc actttccgtc catatagtcc tcttaacagt aatatttgag aggcattttt      60
   attaaagcag tcttaaggag tgttcgtcaa accacatgtt ctgggatcct gagaaagtag     120
   gggaagttta gagaactgaa gctgcacaaa actaatgttt attttctgtt gtgttgtcct     180
   gagaccagct tcttagattg tgt                                             203